# EUROPEAN PATENT APPLICATION

(11) **EP 1 209 231 A1**
(43) Date of publication of application: **29.05.2002**
(21) Application number: 00953507.1
(22) Date of filing: 18.08.2000
(51) Int. Cl.: C12N 15/12, C12N 1/15, C12N 1/19, C12N 1/21, C12N 5/00, C12Q 1/68, C07K 14/47, C07K 16/18, A01K 67/027, G01N 33/53, G01N 33/50

(54) **MEG-1 PROTEIN**

(30) Priority: 19.08.1999 JP 23330199
(71) Applicant: Kurokawa, Kiyoshi, Sinjuku-ku, Tokyo 162-0061 (JP); Miyata, Toshio, Isehara-shi, Kanagawa 259-1132 (JP)
(72) Inventor: MIYATA, Toshio, Isehara-shi Kanagawa 259-1132 (JP)
(74) Representative: Grünecker, Kinkeldey, Stockmair & Schwanhäusser Anwaltssozietät
(86) International application number: JP0005551
(87) International publication number: WO0114552

(57) **Abstract**

The present invention provides a DNA expressed at high frequency in mesangial cells and a protein (Meg-1) encoded by this DNA. These substances are useful in, for example, identifying mesangial cells and detecting abnormalities in mesangial cells. Moreover, the above protein would be helpful for clarification of the functions of mesangial cells and, in its turn, for clarification of the causes of diseases relating to mesangial cells. This protein is expectedly applicable to the treatment, diagnosis, and such of diseases relating to mesangial cells.

SEQUENCE LISTING

<110> MIYATA, TOSHIO
      KUROKAWA, KIYOSHI

<120> Meg-1 protein

<130> KRK-102PCT

<140>
<141>

<150> JP 1999-233301
<151> 1999-08-19

<160> 13

<170> PatentIn Ver. 2.0

<210> 1
<211> 3901
<212> DNA
<213> Homo sapiens

<220>
<221> CDS
<222> (23)..(2872)

<400> 1
gggaggaggg ggcgaccaca ag atg gcg gac ctc tcg ctg ctt cag gag gac 52
                        Met Ala Asp Leu Ser Leu Leu Gln Glu Asp
                          1               5                  10

ctg cag gag gac gca gac gga ttt ggt gtg gat gac tac agc tca gag 100
Leu Gln Glu Asp Ala Asp Gly Phe Gly Val Asp Asp Tyr Ser Ser Glu
                 15                  20                  25

tct gat gtg att att ata cct tca gcc ctg gac ttt gtc tca caa gat 148
Ser Asp Val Ile Ile Ile Pro Ser Ala Leu Asp Phe Val Ser Gln Asp
             30                  35                  40

gaa atg ttg acg ccc ctg ggg aga ttg gac aag tat gct gca agt gag 196
Glu Met Leu Thr Pro Leu Gly Arg Leu Asp Lys Tyr Ala Ala Ser Glu
         45                  50                  55

aac ata ttt aac aga caa atg gtg gcc cgg agt ttg ctc gat acc ttg 244
Asn Ile Phe Asn Arg Gln Met Val Ala Arg Ser Leu Leu Asp Thr Leu
     60                  65                  70

agg gaa gtc tgc gat gat gaa aga gat tgt att gct gtt ttg gaa aga 292
Arg Glu Val Cys Asp Asp Glu Arg Asp Cys Ile Ala Val Leu Glu Arg
75                  80                  85                  90

att agc aga ttg gcc gat gat tca gaa cca act gtg aga gcg gag ctg 340
Ile Ser Arg Leu Ala Asp Asp Ser Glu Pro Thr Val Arg Ala Glu Leu
                95                  100                 105

atg gaa cag gtg cct cac atc gca ctg ttt tgt caa gaa aac cgg cct 388
Met Glu Gln Val Pro His Ile Ala Leu Phe Cys Gln Glu Asn Arg Pro
            110                 115                 120

tca ata cca tat gct ttt tca aaa ttc tta cta cct att gtg gtt aga 436
Ser Ile Pro Tyr Ala Phe Ser Lys Phe Leu Leu Pro Ile Val Val Arg
        125                 130                 135

tac ctt gca gat cag aat aat cag gtg agg aaa aca agt cag gca gct 484
Tyr Leu Ala Asp Gln Asn Asn Gln Val Arg Lys Thr Ser Gln Ala Ala
    140                 145                 150

ttg ctg gct ctg ttg gag cag gag ctc att gaa cga ttt gat gtg gag 532
Leu Leu Ala Leu Leu Glu Gln Glu Leu Ile Glu Arg Phe Asp Val Glu
155                 160                 165                 170

acc aaa gtg tgg cct gtc ctc ata gag ctg aca gcc cca gat agc aat 580
Thr Lys Val Trp Pro Val Leu Ile Glu Leu Thr Ala Pro Asp Ser Asn
                175                 180                 185

gat gat gtg aaa aca gaa gct gtg gct ata atg tgc aaa atg gct ccc 628
Asp Asp Val Lys Thr Glu Ala Val Ala Ile Met Cys Lys Met Ala Pro
            190                 195                 200

atg gtt ggg aag gat att aca gag cgt ctt atc ctc cct agg ttt tgt 676
Met Val Gly Lys Asp Ile Thr Glu Arg Leu Ile Leu Pro Arg Phe Cys
        205                 210                 215

gag atg tgc tgc gat tgc aga atg ttt cac gtt cga aag gtc tgt gct 724
Glu Met Cys Cys Asp Cys Arg Met Phe His Val Arg Lys Val Cys Ala
    220                 225                 230

gcc aat ttt gga gat att tgc agt gta gtt ggc cag caa gct act gaa 772
Ala Asn Phe Gly Asp Ile Cys Ser Val Val Gly Gln Gln Ala Thr Glu
235                 240                 245                 250

gaa atg ttg ctg ccc aga ttt ttc cag ctt tgt tct gat aat gta tgg 820
Glu Met Leu Leu Pro Arg Phe Phe Gln Leu Cys Ser Asp Asn Val Trp
                255                 260                 265

gga gtc cga aag gct tgt gct gaa tgc ttc atg gcg gtt tca tgt gca 868
Gly Val Arg Lys Ala Cys Ala Glu Cys Phe Met Ala Val Ser Cys Ala
            270                 275                 280

aca tgt caa gaa atc cga cgg acc aaa tta tca gca ctt ttt att aat 916
Thr Cys Gln Glu Ile Arg Arg Thr Lys Leu Ser Ala Leu Phe Ile Asn
        285                 290                 295

ttg atc agt gat cct tca cgt tgg gtt cgc caa gca gct ttt cag tct 964
Leu Ile Ser Asp Pro Ser Arg Trp Val Arg Gln Ala Ala Phe Gln Ser
    300                 305                 310

ctg gga cct ttc ata tct act ttt gct aat cca tct agc tca ggc cag 1012
Leu Gly Pro Phe Ile Ser Thr Phe Ala Asn Pro Ser Ser Ser Gly Gln
315                 320                 325                 330

tat ttt aaa gaa gaa agc aaa agt tca gaa gag atg tca gta gaa aac 1060
Tyr Phe Lys Glu Glu Ser Lys Ser Ser Glu Glu Met Ser Val Glu Asn
                335                 340                 345

aaa aat agg acc aga gat caa gaa gcc cca gag gat gta caa gtc agg 1108
Lys Asn Arg Thr Arg Asp Gln Glu Ala Pro Glu Asp Val Gln Val Arg
            350                 355                 360

cca gag gat act cct tca gat ctc agt gtt agt aat tcc agt gtc ata 1156
Pro Glu Asp Thr Pro Ser Asp Leu Ser Val Ser Asn Ser Ser Val Ile
        365                 370                 375

ctg gaa aac acg atg gaa gac cat gct gct gag gca tcc ggg aag cct 1204
Leu Glu Asn Thr Met Glu Asp His Ala Ala Glu Ala Ser Gly Lys Pro
    380                 385                 390

cta ggt gaa att agt gtt cca ctg gac agc tct tta ctt tgt act ttg 1252
Leu Gly Glu Ile Ser Val Pro Leu Asp Ser Ser Leu Leu Cys Thr Leu
395                 400                 405                 410

tcc tca gaa tct cac cag gaa gca gct agt aat gag aat gat aaa aaa 1300
Ser Ser Glu Ser His Gln Glu Ala Ala Ser Asn Glu Asn Asp Lys Lys
                415                 420                 425

cct ggt aac tac aaa tct atg tta cga cca gag gtt ggc acc act tca 1348
Pro Gly Asn Tyr Lys Ser Met Leu Arg Pro Glu Val Gly Thr Thr Ser
            430                 435                 440

caa gat tca gct ctc tta gat cag gaa ttg tat aac tcc ttc cat ttc 1396
Gln Asp Ser Ala Leu Leu Asp Gln Glu Leu Tyr Asn Ser Phe His Phe
        445                 450                 455

tgg agg act cct ctt cct gaa ata gat cta gac ata gag ctt gaa cag 1444
Trp Arg Thr Pro Leu Pro Glu Ile Asp Leu Asp Ile Glu Leu Glu Gln
    460                 465                 470

aac tct ggg gga aaa ccc agc cca gag gga cca gag gaa gaa tct gag 1492
Asn Ser Gly Gly Lys Pro Ser Pro Glu Gly Pro Glu Glu Glu Ser Glu
475                 480                 485                 490

ggc cct gtg ccc agt tct cca aac atc acc atg gcc acc aga aag gaa 1540
Gly Pro Val Pro Ser Ser Pro Asn Ile Thr Met Ala Thr Arg Lys Glu
                495                 500                 505

ctg gaa gaa atg ata gaa aat cta gag ccc cac att gat gat cca gat 1588
Leu Glu Glu Met Ile Glu Asn Leu Glu Pro His Ile Asp Asp Pro Asp
            510                 515                 520

gtt aaa gca caa gtg gaa gtg ctg tcc gct gca cta cgt gct tcc agc 1636
Val Lys Ala Gln Val Glu Val Leu Ser Ala Ala Leu Arg Ala Ser Ser
        525                 530                 535

ctg gat gca cat gaa gag acc atc agt ata gaa aag aga agt gat ttg 1684
Leu Asp Ala His Glu Glu Thr Ile Ser Ile Glu Lys Arg Ser Asp Leu
    540                 545                 550

caa gat gaa ctg gat ata aat gag cta cca aat tgt aaa ata aat caa 1732
Gln Asp Glu Leu Asp Ile Asn Glu Leu Pro Asn Cys Lys Ile Asn Gln
555                 560                 565                 570

gaa gat tct gtg cct tta atc agc gat gct gtt gag aat atg gac tcc 1780
Glu Asp Ser Val Pro Leu Ile Ser Asp Ala Val Glu Asn Met Asp Ser
                575                 580                 585

act ctt cac tat att cac aac gat tca gac ttg agc aac aat agc agt 1828
Thr Leu His Tyr Ile His Asn Asp Ser Asp Leu Ser Asn Asn Ser Ser
            590                 595                 600

ttt agc cct gat gag gaa agg aga act aaa gta caa gat gtt gta cct 1876
Phe Ser Pro Asp Glu Glu Arg Arg Thr Lys Val Gln Asp Val Val Pro
        605                 610                 615

cag gcg ttg tta gat cag tat tta tct atg act gac cct tct cgt gca 1924
Gln Ala Leu Leu Asp Gln Tyr Leu Ser Met Thr Asp Pro Ser Arg Ala
    620                 625                 630

cag acg gtt gac act gaa att gct aag cac tgt gca tat agc ctc cct 1972
Gln Thr Val Asp Thr Glu Ile Ala Lys His Cys Ala Tyr Ser Leu Pro
635                 640                 645                 650

ggt gtg gcc ttg aca ctc gga aga cag aat tgg cac tgc ctg aga gag 2020
Gly Val Ala Leu Thr Leu Gly Arg Gln Asn Trp His Cys Leu Arg Glu
                655                 660                 665

acg tat gag act ctg gcc tca gac atg cag tgg aaa gtt cga cga act 2068
Thr Tyr Glu Thr Leu Ala Ser Asp Met Gln Trp Lys Val Arg Arg Thr
            670                 675                 680

cta gca ttc tcc atc cac gag ctt gca gtt att ctt gga gat caa ttg 2116
Leu Ala Phe Ser Ile His Glu Leu Ala Val Ile Leu Gly Asp Gln Leu
        685                 690                 695

aca gct gca gat ctg gtt cca att ttt aat gga ttt tta aaa gac ctc 2164
Thr Ala Ala Asp Leu Val Pro Ile Phe Asn Gly Phe Leu Lys Asp Leu
    700                 705                 710

gat gaa gtc agg ata ggt gtt ctt aaa cac ttg cat gat ttt ctg aag 2212
Asp Glu Val Arg Ile Gly Val Leu Lys His Leu His Asp Phe Leu Lys
715                 720                 725                 730

ctt ctt cat att gac aaa aga aga gaa tat ctt tat caa ctt cag gag 2260
Leu Leu His Ile Asp Lys Arg Arg Glu Tyr Leu Tyr Gln Leu Gln Glu
                735                 740                 745

ttt ttg gtg aca gat aat agt aga aat tgg cgg ttt cga gct gaa ctg 2308
Phe Leu Val Thr Asp Asn Ser Arg Asn Trp Arg Phe Arg Ala Glu Leu
            750                 755                 760

gct gaa cag ctg att tta ctt cta gag tta tat agt ccc aga gat gtt 2356
Ala Glu Gln Leu Ile Leu Leu Leu Glu Leu Tyr Ser Pro Arg Asp Val
        765                 770                 775

tat gac tat tta cgt ccc att gct ctg aat ctg tgt gca gac aaa gtt 2404
Tyr Asp Tyr Leu Arg Pro Ile Ala Leu Asn Leu Cys Ala Asp Lys Val
    780                 785                 790

tct tct gtt cgt tgg att tcc tac aag ttg gtc agc gag atg gtg aag 2452
Ser Ser Val Arg Trp Ile Ser Tyr Lys Leu Val Ser Glu Met Val Lys
795                 800                 805                 810

aag ctg cac gcg gca aca cca cca acg ttc gga gtg gac ctc atc aat 2500
Lys Leu His Ala Ala Thr Pro Pro Thr Phe Gly Val Asp Leu Ile Asn
                815                 820                 825

gag ctt gtg gag aac ttt ggc aga tgt ccc aag tgg tct ggt cgg caa 2548
Glu Leu Val Glu Asn Phe Gly Arg Cys Pro Lys Trp Ser Gly Arg Gln
            830                 835                 840

gcc ttt gtc ttt gtc tgc cag act gtc att gag gat gac tgc ctt ccc 2596
Ala Phe Val Phe Val Cys Gln Thr Val Ile Glu Asp Asp Cys Leu Pro
        845                 850                 855

atg gac cag ttt gct gtg cat ctc atg ccg cat ctg cta acc tta gca 2644
Met Asp Gln Phe Ala Val His Leu Met Pro His Leu Leu Thr Leu Ala
    860                 865                 870

aat gac agg gtt cct aac gtg cga gtg ctg ctt gca aag aca tta aga 2692
Asn Asp Arg Val Pro Asn Val Arg Val Leu Leu Ala Lys Thr Leu Arg
875                 880                 885                 890

caa act cta cta gaa aaa gac tat ttc ttg gcc tct gcc agc tgc cac 2740
Gln Thr Leu Leu Glu Lys Asp Tyr Phe Leu Ala Ser Ala Ser Cys His
                895                 900                 905

cag gag gct gtg gag cag acc atc atg gct ctt cag atg gac cgt gac 2788
Gln Glu Ala Val Glu Gln Thr Ile Met Ala Leu Gln Met Asp Arg Asp
            910                 915                 920

agc gat gtc aag tat ttt gca agc atc cac cct gcc agt acc aaa atc 2836
Ser Asp Val Lys Tyr Phe Ala Ser Ile His Pro Ala Ser Thr Lys Ile
        925                 930                 935

tcc gaa gat gcc atg agc aca gcg tcc tca acc tac tagaaggctt 2882
Ser Glu Asp Ala Met Ser Thr Ala Ser Ser Thr Tyr
    940                 945                 950

gaatctcggt gtctttcctg cttccatgag agccgaggtt cagtgggcat tcgccacgca 2942

tgtgacctgg gatagctttc gggggaggag agaccttcct ctcctgcgga cttcattgca 3002

ggtgcaagtt gcctacaccc aataccaggg atttcaagag tcaagagaaa gtacagtaaa 3062

cactattatc ttatcttgac tttaagggga aataatttct cagaggatta taattgtcac 3122

cgaagcctta aatccttctc ttcctgactg aatgaaactt gaattggcag agcattttcc 3182

ttatggaagg gatgagattc ccagagacct gcattgcttt ctcctggttt tatttaacaa 3242

tcgacaaatg aaattcttac agcctgaagg cagacgtgtg cccagatgtg aaagagacct 3302

tcagtatcag ccctaactct tctctcccag gaaggacttg ctgggctctg tggccagctg 3362

tccagcccag ccctgtgtgt gaatcgtttg tgacgtgtgc aaatgggaaa ggaggggttt 3422

ttacatctcc taaaggacct gatgccaaca caagtaggat tgacttaaac tcttaagcgc 3482

agcatattgc tgtacacatt tacagaatgg ttgctgagtg tctgtgtctg attttttcat 3542

gctggtcatg acctgaagga aatttattag acgtataatg tatgtctggt gtttttaact 3602

tgatcatgat cagctctgag gtgcaacttc ttcacatact gtacatacct gtgaccactc 3662

ttgggagtgc tgcagtcttt aatcatgctg tttaaactgt tgtggcacaa gttctcttgt 3722

ccaaataaaa tttattaata agatctatag agagagatat atacactttt gattgttttc 3782

tagatgtcta ccaataaatg caatttgtga cctgtattaa tgatttaaag tggggaaact 3842

agattaaaat atttgtcttt taaaaaaata aaaaaaaaaa aaaaaaaaaa aaaaaaaaa 3901

<210> 2
<211> 950
<212> PRT
<213> Homo sapiens

<400> 2
Met Ala Asp Leu Ser Leu Leu Gln Glu Asp Leu Gln Glu Asp Ala Asp
1               5                   10                  15

Gly Phe Gly Val Asp Asp Tyr Ser Ser Glu Ser Asp Val Ile Ile Ile
            20                  25                  30

Pro Ser Ala Leu Asp Phe Val Ser Gln Asp Glu Met Leu Thr Pro Leu
        35                  40                  45

Gly Arg Leu Asp Lys Tyr Ala Ala Ser Glu Asn Ile Phe Asn Arg Gln
    50                  55                  60

Met Val Ala Arg Ser Leu Leu Asp Thr Leu Arg Glu Val Cys Asp Asp
65                  70                  75                  80

Glu Arg Asp Cys Ile Ala Val Leu Glu Arg Ile Ser Arg Leu Ala Asp
                85                  90                  95

Asp Ser Glu Pro Thr Val Arg Ala Glu Leu Met Glu Gln Val Pro His
            100                 105                 110

Ile Ala Leu Phe Cys Gln Glu Asn Arg Pro Ser Ile Pro Tyr Ala Phe
        115                 120                 125

Ser Lys Phe Leu Leu Pro Ile Val Val Arg Tyr Leu Ala Asp Gln Asn
    130                 135                 140

Asn Gln Val Arg Lys Thr Ser Gln Ala Ala Leu Leu Ala Leu Leu Glu
145                 150                 155                 160

Gln Glu Leu Ile Glu Arg Phe Asp Val Glu Thr Lys Val Trp Pro Val
                165                 170                 175

Leu Ile Glu Leu Thr Ala Pro Asp Ser Asn Asp Asp Val Lys Thr Glu
            180                 185                 190

Ala Val Ala Ile Met Cys Lys Met Ala Pro Met Val Gly Lys Asp Ile
        195                 200                 205

Thr Glu Arg Leu Ile Leu Pro Arg Phe Cys Glu Met Cys Cys Asp Cys
    210                 215                 220

Arg Met Phe His Val Arg Lys Val Cys Ala Ala Asn Phe Gly Asp Ile
225                 230                 235                 240

Cys Ser Val Val Gly Gln Gln Ala Thr Glu Glu Met Leu Leu Pro Arg
                245                 250                 255

Phe Phe Gln Leu Cys Ser Asp Asn Val Trp Gly Val Arg Lys Ala Cys
            260                 265                 270

Ala Glu Cys Phe Met Ala Val Ser Cys Ala Thr Cys Gln Glu Ile Arg
        275                 280                 285

Arg Thr Lys Leu Ser Ala Leu Phe Ile Asn Leu Ile Ser Asp Pro Ser
    290                 295                 300

Arg Trp Val Arg Gln Ala Ala Phe Gln Ser Leu Gly Pro Phe Ile Ser
305                 310                 315                 320

Thr Phe Ala Asn Pro Ser Ser Ser Gly Gln Tyr Phe Lys Glu Glu Ser
                325                 330                 335

Lys Ser Ser Glu Glu Met Ser Val Glu Asn Lys Asn Arg Thr Arg Asp
            340                 345                 350

Gln Glu Ala Pro Glu Asp Val Gln Val Arg Pro Glu Asp Thr Pro Ser
        355                 360                 365

Asp Leu Ser Val Ser Asn Ser Ser Val Ile Leu Glu Asn Thr Met Glu
    370                 375                 380

Asp His Ala Ala Glu Ala Ser Gly Lys Pro Leu Gly Glu Ile Ser Val
385                 390                 395                 400

Pro Leu Asp Ser Ser Leu Leu Cys Thr Leu Ser Ser Glu Ser His Gln
                405                 410                 415

Glu Ala Ala Ser Asn Glu Asn Asp Lys Lys Pro Gly Asn Tyr Lys Ser
            420                 425                 430

Met Leu Arg Pro Glu Val Gly Thr Thr Ser Gln Asp Ser Ala Leu Leu
        435                 440                 445

Asp Gln Glu Leu Tyr Asn Ser Phe His Phe Trp Arg Thr Pro Leu Pro
    450                 455                 460

Glu Ile Asp Leu Asp Ile Glu Leu Glu Gln Asn Ser Gly Gly Lys Pro
465                 470                 475                 480

Ser Pro Glu Gly Pro Glu Glu Glu Ser Glu Gly Pro Val Pro Ser Ser
                485                 490                 495

Pro Asn Ile Thr Met Ala Thr Arg Lys Glu Leu Glu Glu Met Ile Glu
            500                 505                 510

Asn Leu Glu Pro His Ile Asp Asp Pro Asp Val Lys Ala Gln Val Glu
        515                 520                 525

Val Leu Ser Ala Ala Leu Arg Ala Ser Ser Leu Asp Ala His Glu Glu
    530                 535                 540

Thr Ile Ser Ile Glu Lys Arg Ser Asp Leu Gln Asp Glu Leu Asp Ile
545                 550                 555                 560

Asn Glu Leu Pro Asn Cys Lys Ile Asn Gln Glu Asp Ser Val Pro Leu
                565                 570                 575

Ile Ser Asp Ala Val Glu Asn Met Asp Ser Thr Leu His Tyr Ile His
            580                 585                 590

Asn Asp Ser Asp Leu Ser Asn Asn Ser Ser Phe Ser Pro Asp Glu Glu
        595                 600                 605

Arg Arg Thr Lys Val Gln Asp Val Val Pro Gln Ala Leu Leu Asp Gln
    610                 615                 620

Tyr Leu Ser Met Thr Asp Pro Ser Arg Ala Gln Thr Val Asp Thr Glu
625                 630                 635                 640

Ile Ala Lys His Cys Ala Tyr Ser Leu Pro Gly Val Ala Leu Thr Leu
                645                 650                 655

Gly Arg Gln Asn Trp His Cys Leu Arg Glu Thr Tyr Glu Thr Leu Ala
            660                 665                 670

Ser Asp Met Gln Trp Lys Val Arg Arg Thr Leu Ala Phe Ser Ile His
        675                 680                 685

Glu Leu Ala Val Ile Leu Gly Asp Gln Leu Thr Ala Ala Asp Leu Val
    690                 695                 700

Pro Ile Phe Asn Gly Phe Leu Lys Asp Leu Asp Glu Val Arg Ile Gly
705                 710                 715                 720

Val Leu Lys His Leu His Asp Phe Leu Lys Leu Leu His Ile Asp Lys
                725                 730                 735

Arg Arg Glu Tyr Leu Tyr Gln Leu Gln Glu Phe Leu Val Thr Asp Asn
            740                 745                 750

Ser Arg Asn Trp Arg Phe Arg Ala Glu Leu Ala Glu Gln Leu Ile Leu
        755                 760                 765

Leu Leu Glu Leu Tyr Ser Pro Arg Asp Val Tyr Asp Tyr Leu Arg Pro
    770                 775                 780

Ile Ala Leu Asn Leu Cys Ala Asp Lys Val Ser Ser Val Arg Trp Ile
785                 790                 795                 800

Ser Tyr Lys Leu Val Ser Glu Met Val Lys Lys Leu His Ala Ala Thr
                805                 810                 815

Pro Pro Thr Phe Gly Val Asp Leu Ile Asn Glu Leu Val Glu Asn Phe
            820                 825                 830

Gly Arg Cys Pro Lys Trp Ser Gly Arg Gln Ala Phe Val Phe Val Cys
        835                 840                 845

Gln Thr Val Ile Glu Asp Asp Cys Leu Pro Met Asp Gln Phe Ala Val
    850                 855                 860

His Leu Met Pro His Leu Leu Thr Leu Ala Asn Asp Arg Val Pro Asn
865                 870                 875                 880

Val Arg Val Leu Leu Ala Lys Thr Leu Arg Gln Thr Leu Leu Glu Lys
                885                 890                 895

Asp Tyr Phe Leu Ala Ser Ala Ser Cys His Gln Glu Ala Val Glu Gln
            900                 905                 910

Thr Ile Met Ala Leu Gln Met Asp Arg Asp Ser Asp Val Lys Tyr Phe
        915                 920                 925

Ala Ser Ile His Pro Ala Ser Thr Lys Ile Ser Glu Asp Ala Met Ser
    930                 935                 940

Thr Ala Ser Ser Thr Tyr
945                 950


<210> 3
<211> 18
<212> DNA
<213> Artificial Sequence

<220>
<223> Description of Artificial Sequence:Artificially
      Synthesized Primer Sequence

<400> 3
tgtaaaacga cggccagt                                                18


<210> 4
<211> 21
<212> DNA
<213> Artificial Sequence

<220>
<223> Description of Artificial Sequence:Artificially
      Synthesized Primer Sequence

<400> 4
accatgatta cgccaagctt g                                            21


<210> 5
<211> 15
<212> DNA
<213> Artificial Sequence

<220>
<223> Description of Artificial Sequence:Artificially
      Synthesized Primer Sequence

<220>
<223> 5'-phosphorylation

<400> 5
tcagagaggt cattc                                                        15


<210> 6
<211> 18
<212> DNA
<213> Artificial Sequence

<220>
<223> Description of Artificial Sequence:Artificially
      Synthesized Primer Sequence

<400> 6
tcattgatgg gtcctcaa                                                     18


<210> 7
<211> 18
<212> DNA
<213> Artificial Sequence

<220>
<223> Description of Artificial Sequence:Artificially
      Synthesized Primer Sequence

<400> 7
agattcttga gctcagat                                                     18


<210> 8
<211> 18
<212> DNA
<213> Artificial Sequence

<220>
<223> Description of Artificial Sequence:Artificially
      Synthesized Primer Sequence

<400> 8
aatggtggca taaacatg                                                     18


<210> 9
<211> 18
<212> DNA
<213> Artificial Sequence

<220>
<223> Description of Artificial Sequence:Artificially
      Synthesized Primer Sequence

<400> 9
acagacaaat tgaacttc                                                    18


<210> 10
<211> 34
<212> DNA
<213> Artificial Sequence

<220>
<223> Description of Artificial Sequence:Artificially
      Synthesized Probe Sequence

<400> 10
ctcatggaag caggaaagac accgagattc aagc                                  34


<210> 11
<211> 3568
<212> DNA
<213> RATTUS NORVEGICUS

<220>
<221> CDS
<222> (7)..(2862)

<400> 11
cacaag atg gcg gac ctc tcg ctg ctc cag gag gac ctg ccg gag gac        48
       Met Ala Asp Leu Ser Leu Leu Gln Glu Asp Leu Pro Glu Asp
       1               5                   10

gcg gac gga ctt ggt gtg gat gac tac agc tca gag tct gat gtg att       96
Ala Asp Gly Leu Gly Val Asp Asp Tyr Ser Ser Glu Ser Asp Val Ile
15                  20                  25                  30

att ata cct tca gcc ctg gac ttc gtc tca caa gat gaa atg ttg aca      144
Ile Ile Pro Ser Ala Leu Asp Phe Val Ser Gln Asp Glu Met Leu Thr
                35                  40                  45

ccc ttg ggg agg ctg gac aag tat gct gca agt gag aac gtc ttt aac      192
Pro Leu Gly Arg Leu Asp Lys Tyr Ala Ala Ser Glu Asn Val Phe Asn
            50                  55                  60

aga caa atg gtg gcc cgg agt ttg ctg gat act ctg agg gaa gtc tgt 240
Arg Gln Met Val Ala Arg Ser Leu Leu Asp Thr Leu Arg Glu Val Cys
        65                  70                  75

ggt gag gag aga gac tgc att gct gtc ttg gaa agg atc agc cga ttg 288
Gly Glu Glu Arg Asp Cys Ile Ala Val Leu Glu Arg Ile Ser Arg Leu
    80                  85                  90

gct gat gac tca gaa cca acc gtg aga gcc gag ctg atg gaa cag gtg 336
Ala Asp Asp Ser Glu Pro Thr Val Arg Ala Glu Leu Met Glu Gln Val
95                  100                 105                 110

ccg cac atc gca ctg ttt tgt caa gag aac cga cct tcc ata cca tat 384
Pro His Ile Ala Leu Phe Cys Gln Glu Asn Arg Pro Ser Ile Pro Tyr
                115                 120                 125

gcc ttt tcc aag tac tta ctg cca atc gtg gtt aga tac ctt gca gac 432
Ala Phe Ser Lys Tyr Leu Leu Pro Ile Val Val Arg Tyr Leu Ala Asp
            130                 135                 140

cag aat aac cag gtg agg aaa acc agc cag gca gct ttg ctg gct ctg 480
Gln Asn Asn Gln Val Arg Lys Thr Ser Gln Ala Ala Leu Leu Ala Leu
        145                 150                 155

ctg gag cag gag ctg att gag cga ctc gat gtg gag acc aag gtg tgc 528
Leu Glu Gln Glu Leu Ile Glu Arg Leu Asp Val Glu Thr Lys Val Cys
    160                 165                 170

ccc gtc ctc ata gac ttg act gcc cca gac agc aat gac gat gtg aag 576
Pro Val Leu Ile Asp Leu Thr Ala Pro Asp Ser Asn Asp Asp Val Lys
175                 180                 185                 190

aca gag gcc gtg gct ata atg tgc aag atg gcc ccc atg gtt ggg aaa 624
Thr Glu Ala Val Ala Ile Met Cys Lys Met Ala Pro Met Val Gly Lys
                195                 200                 205

gat att aca gag cgt ctc atc ctc cct agg ttt tgt gag atg tgc tgt 672
Asp Ile Thr Glu Arg Leu Ile Leu Pro Arg Phe Cys Glu Met Cys Cys
            210                 215                 220

gac tgt aga atg ttt cac gtc cga aag gtc tgt gct gcc aat ttt gga 720
Asp Cys Arg Met Phe His Val Arg Lys Val Cys Ala Ala Asn Phe Gly
        225                 230                 235

gac att tgc agc gta gtt ggc cag caa gct aca gaa gaa atg ctg ctg 768
Asp Ile Cys Ser Val Val Gly Gln Gln Ala Thr Glu Glu Met Leu Leu
    240                 245                 250

ccc agg ttc ttc cag ctg tgt tct gac aat gtg tgg ggc gtc cgg aag 816
Pro Arg Phe Phe Gln Leu Cys Ser Asp Asn Val Trp Gly Val Arg Lys
255                 260                 265                 270

gcc tgt gct gag tgc ttc atg gcc gtc tcc tgc gcg aca tgc caa gaa 864
Ala Cys Ala Glu Cys Phe Met Ala Val Ser Cys Ala Thr Cys Gln Glu
                275                 280                 285

atc cga cgg aca aag ttg tca gca ctg ttt att aac ttg atc agt gat 912
Ile Arg Arg Thr Lys Leu Ser Ala Leu Phe Ile Asn Leu Ile Ser Asp
            290                 295                 300

cct tca cgt tgg gtt cgc caa gca gcc ttt cag tcc ctg ggg cct ttc 960
Pro Ser Arg Trp Val Arg Gln Ala Ala Phe Gln Ser Leu Gly Pro Phe
        305                 310                 315

ata tcc aca ttt gct aat cca tca agc tcg ggc cag tgc ttc aaa gat 1008
Ile Ser Thr Phe Ala Asn Pro Ser Ser Ser Gly Gln Cys Phe Lys Asp
    320                 325                 330

gag agc aaa agc tca gaa gac aaa gac agg atc aga gac gat ggt gtt 1056
Glu Ser Lys Ser Ser Glu Asp Lys Asp Arg Ile Arg Asp Asp Gly Val
335                 340                 345                 350

gta caa gaa gag cag agc agg cca gag gac gca cct tca gac ctc agt 1104
Val Gln Glu Glu Gln Ser Arg Pro Glu Asp Ala Pro Ser Asp Leu Ser
                355                 360                 365

gcc cct cac tcc agt gcc agg ctg gac ggc aca ctt gaa ggc tgt gct 1152
Ala Pro His Ser Ser Ala Arg Leu Asp Gly Thr Leu Glu Gly Cys Ala
            370                 375                 380

gcc gag acg cct ggg gac tct gca ggt gac atg cgt gtt cca gcg gac 1200
Ala Glu Thr Pro Gly Asp Ser Ala Gly Asp Met Arg Val Pro Ala Asp
        385                 390                 395

agc tcc tta ctc tgt act ttg tcc tca gag tct cct cag gaa gca gct 1248
Ser Ser Leu Leu Cys Thr Leu Ser Ser Glu Ser Pro Gln Glu Ala Ala
    400                 405                 410

agt gac gct gag agt ggt aaa aag cac gat aac aac agc aag tct gcg 1296
Ser Asp Ala Glu Ser Gly Lys Lys His Asp Asn Asn Ser Lys Ser Ala
415                 420                 425                 430

tcc cgg cca gac gtt ggc acc agc tcc cca gag ccc act ccc tta gat 1344
Ser Arg Pro Asp Val Gly Thr Ser Ser Pro Glu Pro Thr Pro Leu Asp
                435                 440                 445

cag gaa atg ttc aac tcc ttc cat ttc tgg agg act cct cta ccc cag 1392
Gln Glu Met Phe Asn Ser Phe His Phe Trp Arg Thr Pro Leu Pro Gln
            450                 455                 460

ata gat ctt gat aaa gag ctc caa cag gac cct ggg gag agg ccc agc 1440
Ile Asp Leu Asp Lys Glu Leu Gln Gln Asp Pro Gly Glu Arg Pro Ser
        465                 470                 475

cca gag aga aca gga gat gca cct gca gcc cct gta cca ggt tct ccc 1488
Pro Glu Arg Thr Gly Asp Ala Pro Ala Ala Pro Val Pro Gly Ser Pro
    480                 485                 490

agt atc acc atg gct acc cgg aag gaa cta gaa gaa atg ata gaa aac 1536
Ser Ile Thr Met Ala Thr Arg Lys Glu Leu Glu Glu Met Ile Glu Asn
495                 500                 505                 510

cta gag ccg cac atg gat gac ccg gat gtt aaa gcc cag gtg gaa gtg 1584
Leu Glu Pro His Met Asp Asp Pro Asp Val Lys Ala Gln Val Glu Val
                515                 520                 525

ctg tcg gcc gcc ctg cgc gct tct acc ctg gat gct cac gac gag gct 1632
Leu Ser Ala Ala Leu Arg Ala Ser Thr Leu Asp Ala His Asp Glu Ala
            530                 535                 540

ggc ggt gca gag cag cgg agt gag ctg cag gac gac gca gtg ggt gcc 1680
Gly Gly Ala Glu Gln Arg Ser Glu Leu Gln Asp Asp Ala Val Gly Ala
        545                 550                 555

ggc ggc gag ctt cca aac tgt agc atc agc gaa gac act tct gag cct 1728
Gly Gly Glu Leu Pro Asn Cys Ser Ile Ser Glu Asp Thr Ser Glu Pro
    560                 565                 570

ctg gtc atc gct gct gag gag aat atg gag gcc act cct gac tat atc 1776
Leu Val Ile Ala Ala Glu Glu Asn Met Glu Ala Thr Pro Asp Tyr Ile
575                 580                 585                 590

cat gga ggt gcg gat gta ggc ccc ggt ggc ggt ggt ggc ttc agc ccg 1824
His Gly Gly Ala Asp Val Gly Pro Gly Gly Gly Gly Gly Phe Ser Pro
                595                 600                 605

gat gaa gag agg aga ccc aaa gtc cag gat gtc gta cca caa gcg tta 1872
Asp Glu Glu Arg Arg Pro Lys Val Gln Asp Val Val Pro Gln Ala Leu
            610                 615                 620

cta gac cag tac ctg tca atg acc gac cct tct cga gca cag aca gtc 1920
Leu Asp Gln Tyr Leu Ser Met Thr Asp Pro Ser Arg Ala Gln Thr Val
        625                 630                 635

gac acc gag atc gct aag cac tgt gca tac agt ctg ccg ggt gtg gct 1968
Asp Thr Glu Ile Ala Lys His Cys Ala Tyr Ser Leu Pro Gly Val Ala
    640                 645                 650

ctg acc ctt ggc aga cag aac tgg cac tgc ttg aga gag act tac gag 2016
Leu Thr Leu Gly Arg Gln Asn Trp His Cys Leu Arg Glu Thr Tyr Glu
655                 660                 665                 670

acc cta gcg tca gac atg cag tgg aaa gtt cga aga act ctg gcc ttc 2064
Thr Leu Ala Ser Asp Met Gln Trp Lys Val Arg Arg Thr Leu Ala Phe
                675                 680                 685

tcc atc cat gag ctc gcg gtg att ctc ggg gac cag ctg aca gca gca 2112
Ser Ile His Glu Leu Ala Val Ile Leu Gly Asp Gln Leu Thr Ala Ala
            690                 695                 700

gac ctg gtt ccg att ttt aat ggg ttt tta aaa gat ctt gac gaa gtc 2160
Asp Leu Val Pro Ile Phe Asn Gly Phe Leu Lys Asp Leu Asp Glu Val
        705                 710                 715

agg ata ggt gtt ctc aaa cac ttg cat gac ttt ctg aag ctt ctt cat 2208
Arg Ile Gly Val Leu Lys His Leu His Asp Phe Leu Lys Leu Leu His
    720                 725                 730

att gat aaa aga aga gag tac ctt tat caa ctc cag gag ttt ttg gtg 2256
Ile Asp Lys Arg Arg Glu Tyr Leu Tyr Gln Leu Gln Glu Phe Leu Val
735                 740                 745                 750

aca gac aac agt aga aat tgg cgg ttt cga gct gaa ctg gca gaa cag 2304
Thr Asp Asn Ser Arg Asn Trp Arg Phe Arg Ala Glu Leu Ala Glu Gln
                755                 760                 765

ctg att tta ctt cta gaa tta tat agt ccc aga gat gtt tat gat tac 2352
Leu Ile Leu Leu Leu Glu Leu Tyr Ser Pro Arg Asp Val Tyr Asp Tyr
            770                 775                 780

tta cgt ccc att gct ctg aat ctg tgt gca gac aaa gtt tct tca gtc 2400
Leu Arg Pro Ile Ala Leu Asn Leu Cys Ala Asp Lys Val Ser Ser Val
        785                 790                 795

cgt tgg att tcc tac aag ttg gtc agt gag atg gtg aag aag cta cac 2448
Arg Trp Ile Ser Tyr Lys Leu Val Ser Glu Met Val Lys Lys Leu His
    800                 805                 810

atg gcg acg ccg cca acg ttc gga gtc gag ctc atc aat gag ctg gtg 2496
Met Ala Thr Pro Pro Thr Phe Gly Val Glu Leu Ile Asn Glu Leu Val
815                 820                 825                 830

gag aac ttc ggc agg tgt cca aag tgg tcg ggc cgg cag gcc ttc gtc     2544
Glu Asn Phe Gly Arg Cys Pro Lys Trp Ser Gly Arg Gln Ala Phe Val
                835                 840                 845

ttc gtg tgc cag act gtc att gag gac gac tgc ctc ccc atg gac cag     2592
Phe Val Cys Gln Thr Val Ile Glu Asp Asp Cys Leu Pro Met Asp Gln
            850                 855                 860

ttt gct gtg cac ctg atg cca cat ttg ctg acc ttg gca aat gac agg     2640
Phe Ala Val His Leu Met Pro His Leu Leu Thr Leu Ala Asn Asp Arg
        865                 870                 875

gtt ccc aac gtt aga gtg ctg ctt gca aaa acc ctt cga cag act cta     2688
Val Pro Asn Val Arg Val Leu Leu Ala Lys Thr Leu Arg Gln Thr Leu
    880                 885                 890

cta gag aaa gaa tac ttc tta gcc tct gcc agc tgt cat cag gag gcc     2736
Leu Glu Lys Glu Tyr Phe Leu Ala Ser Ala Ser Cys His Gln Glu Ala
895                 900                 905                 910

gtg gag cag aca atc atg gcc ctt cag atg gat cga gac agt gac gtc     2784
Val Glu Gln Thr Ile Met Ala Leu Gln Met Asp Arg Asp Ser Asp Val
                915                 920                 925

aag tac ttt gca agc atc cac ccg tcc agt acc aaa ctc tct gaa gac     2832
Lys Tyr Phe Ala Ser Ile His Pro Ser Ser Thr Lys Leu Ser Glu Asp
            930                 935                 940

gca atg agt aca gct tcc tcc acc tac tga cccctgaccc acggtgtcct     2882
Ala Met Ser Thr Ala Ser Ser Thr Tyr
        945                 950

tcctgcatcc gcgagagcct ggcctcagcc gcctgcgcca ctcgggacag ctgtggtggt     2942
ggggcctccc tcctgccagc tcattcgcag gtgcaagttg cctactccca taccagtggt     3002
tttaagagtc aagagaaagt acagtaaaca ctattatctt atcttgactt agggaaagta     3062
aactctcaga ggattataat tgtcaccaaa gccttaactc attacttcct cttcctgact     3122
gaatgacttg aattggcaga gcattttccc ttcgggaagg aggargttcc cagagacctg     3182
cgctgctttc tcctggtttt atttaacgnt ggtaaatggc attcttaccg ccggaaggtg     3242
gacacgcacc ggacagggag gcctgggtat tagcccaagc acttgtccca ggtgcgagtc     3302
tgctcggctc ntgggctgcc ctgcccagcc ctaagtgtga atagttcttg gcgtgtataa     3362
atgacaggag tttttcctct cctaagggct tgatgttaac actaagtaga atntgatttt     3422
gactcctaat gcagcacatt gctgtacaca tttacagaat gtttgcagac tctctcccga     3482
ctcttttcat gctggtcatg acgtgaaggg gacttctcag cagatattgt gtcaggtgtt     3542
cttaacttga tcatggtcag ctctgaggtg cgactttcct tcccatgctg cacacccctg     3602
tggccacgct ggggcatgca gccttaatca tgctgttaga actgttgtgg cacaag     3658

<210> 12

<211> 951
<212> DNA
<213> RATTUS NORVEGICUS

<400> 12
Met Ala Asp Leu Ser Leu Leu Gln Glu Asp Leu Pro Glu Asp Ala Asp
1               5                   10                  15

Gly Leu Gly Val Asp Asp Tyr Ser Ser Glu Ser Asp Val Ile Ile Ile
            20                  25                  30

Pro Ser Ala Leu Asp Phe Val Ser Gln Asp Glu Met Leu Thr Pro Leu
        35                  40                  45

Gly Arg Leu Asp Lys Tyr Ala Ala Ser Glu Asn Val Phe Asn Arg Gln
    50                  55                  60

Met Val Ala Arg Ser Leu Leu Asp Thr Leu Arg Glu Val Cys Gly Glu
65                  70                  75                  80

Glu Arg Asp Cys Ile Ala Val Leu Glu Arg Ile Ser Arg Leu Ala Asp
                85                  90                  95

Asp Ser Glu Pro Thr Val Arg Ala Glu Leu Met Glu Gln Val Pro His
            100                 105                 110

Ile Ala Leu Phe Cys Gln Glu Asn Arg Pro Ser Ile Pro Tyr Ala Phe
        115                 120                 125

Ser Lys Tyr Leu Leu Pro Ile Val Val Arg Tyr Leu Ala Asp Gln Asn
    130                 135                 140

Asn Gln Val Arg Lys Thr Ser Gln Ala Ala Leu Leu Ala Leu Leu Glu
145                 150                 155                 160

Gln Glu Leu Ile Glu Arg Leu Asp Val Glu Thr Lys Val Cys Pro Val
                165                 170                 175

Leu Ile Asp Leu Thr Ala Pro Asp Ser Asn Asp Asp Val Lys Thr Glu
            180                 185                 190

Ala Val Ala Ile Met Cys Lys Met Ala Pro Met Val Gly Lys Asp Ile
        195                 200                 205

Thr Glu Arg Leu Ile Leu Pro Arg Phe Cys Glu Met Cys Cys Asp Cys
    210                 215                 220

Arg Met Phe His Val Arg Lys Val Cys Ala Ala Asn Phe Gly Asp Ile

225                 230                 235                 240

Cys Ser Val Val Gly Gln Gln Ala Thr Glu Glu Met Leu Leu Pro Arg
                245                 250                 255

Phe Phe Gln Leu Cys Ser Asp Asn Val Trp Gly Val Arg Lys Ala Cys
            260                 265                 270

Ala Glu Cys Phe Met Ala Val Ser Cys Ala Thr Cys Gln Glu Ile Arg
        275                 280                 285

Arg Thr Lys Leu Ser Ala Leu Phe Ile Asn Leu Ile Ser Asp Pro Ser
    290                 295                 300

Arg Trp Val Arg Gln Ala Ala Phe Gln Ser Leu Gly Pro Phe Ile Ser
305                 310                 315                 320

Thr Phe Ala Asn Pro Ser Ser Ser Gly Gln Cys Phe Lys Asp Glu Ser
                325                 330                 335

Lys Ser Ser Glu Asp Lys Asp Arg Ile Arg Asp Asp Gly Val Val Gln
            340                 345                 350

Glu Glu Gln Ser Arg Pro Glu Asp Ala Pro Ser Asp Leu Ser Ala Pro
        355                 360                 365

His Ser Ser Ala Arg Leu Asp Gly Thr Leu Glu Gly Cys Ala Ala Glu
    370                 375                 380

Thr Pro Gly Asp Ser Ala Gly Asp Met Arg Val Pro Ala Asp Ser Ser
385                 390                 395                 400

Leu Leu Cys Thr Leu Ser Ser Glu Ser Pro Gln Glu Ala Ala Ser Asp
                405                 410                 415

Ala Glu Ser Gly Lys Lys His Asp Asn Asn Ser Lys Ser Ala Ser Arg
            420                 425                 430

Pro Asp Val Gly Thr Ser Ser Pro Glu Pro Thr Pro Leu Asp Gln Glu
        435                 440                 445

Met Phe Asn Ser Phe His Phe Trp Arg Thr Pro Leu Pro Gln Ile Asp
    450                 455                 460

Leu Asp Lys Glu Leu Gln Gln Asp Pro Gly Glu Arg Pro Ser Pro Glu
465                 470                 475                 480

Arg Thr Gly Asp Ala Pro Ala Ala Pro Val Pro Gly Ser Pro Ser Ile

                485                 490                 495

Thr Met Ala Thr Arg Lys Glu Leu Glu Glu Met Ile Glu Asn Leu Glu
            500                 505                 510

Pro His Met Asp Asp Pro Asp Val Lys Ala Gln Val Glu Val Leu Ser
        515                 520                 525

Ala Ala Leu Arg Ala Ser Thr Leu Asp Ala His Asp Glu Ala Gly Gly
    530                 535                 540

Ala Glu Gln Arg Ser Glu Leu Gln Asp Asp Ala Val Gly Ala Gly Gly
545                 550                 555                 560

Glu Leu Pro Asn Cys Ser Ile Ser Glu Asp Thr Ser Glu Pro Leu Val
                565                 570                 575

Ile Ala Ala Glu Glu Asn Met Glu Ala Thr Pro Asp Tyr Ile His Gly
            580                 585                 590

Gly Ala Asp Val Gly Pro Gly Gly Gly Gly Gly Phe Ser Pro Asp Glu
        595                 600                 605

Glu Arg Arg Pro Lys Val Gln Asp Val Val Pro Gln Ala Leu Leu Asp
    610                 615                 620

Gln Tyr Leu Ser Met Thr Asp Pro Ser Arg Ala Gln Thr Val Asp Thr
625                 630                 635                 640

Glu Ile Ala Lys His Cys Ala Tyr Ser Leu Pro Gly Val Ala Leu Thr
                645                 650                 655

Leu Gly Arg Gln Asn Trp His Cys Leu Arg Glu Thr Tyr Glu Thr Leu
            660                 665                 670

Ala Ser Asp Met Gln Trp Lys Val Arg Arg Thr Leu Ala Phe Ser Ile
        675                 680                 685

His Glu Leu Ala Val Ile Leu Gly Asp Gln Leu Thr Ala Ala Asp Leu
    690                 695                 700

Val Pro Ile Phe Asn Gly Phe Leu Lys Asp Leu Asp Glu Val Arg Ile
705                 710                 715                 720

Gly Val Leu Lys His Leu His Asp Phe Leu Lys Leu Leu His Ile Asp
                725                 730                 735

Lys Arg Arg Glu Tyr Leu Tyr Gln Leu Gln Glu Phe Leu Val Thr Asp

740                 745                 750

Asn Ser Arg Asn Trp Arg Phe Arg Ala Glu Leu Ala Glu Gln Leu Ile
        755                 760                 765

Leu Leu Leu Glu Leu Tyr Ser Pro Arg Asp Val Tyr Asp Tyr Leu Arg
    770                 775                 780

Pro Ile Ala Leu Asn Leu Cys Ala Asp Lys Val Ser Ser Val Arg Trp
785                 790                 795                 800

Ile Ser Tyr Lys Leu Val Ser Glu Met Val Lys Lys Leu His Met Ala
                805                 810                 815

Thr Pro Pro Thr Phe Gly Val Glu Leu Ile Asn Glu Leu Val Glu Asn
            820                 825                 830

Phe Gly Arg Cys Pro Lys Trp Ser Gly Arg Gln Ala Phe Val Phe Val
        835                 840                 845

Cys Gln Thr Val Ile Glu Asp Asp Cys Leu Pro Met Asp Gln Phe Ala
    850                 855                 860

Val His Leu Met Pro His Leu Leu Thr Leu Ala Asn Asp Arg Val Pro
865                 870                 875                 880

Asn Val Arg Val Leu Leu Ala Lys Thr Leu Arg Gln Thr Leu Leu Glu
                885                 890                 895

Lys Glu Tyr Phe Leu Ala Ser Ala Ser Cys His Gln Glu Ala Val Glu
            900                 905                 910

Gln Thr Ile Met Ala Leu Gln Met Asp Arg Asp Ser Asp Val Lys Tyr
        915                 920                 925

Phe Ala Ser Ile His Pro Ser Ser Thr Lys Leu Ser Glu Asp Ala Met
    930                 935                 940

Ser Thr Ala Ser Ser Thr Tyr
945                 950

<210> 13
<211> 1132
<212> DNA
<213> RATTUS NORVEGICUS

<400> 13
gtctcctgcg cgacatgcca agaaatccga cggacaaagt tgtcagcact gtttattaac      60

ttgatcagtg atccttcacg ttgggttcgc caagcagcct ttcagtccct ggggcctttc 120
atatccacat ttgctaatcc atcaagctcg ggccagtgct tcaaagatga gagcaaaagc 180
tcagaagaca aagacaggat cagagacgat ggtgttgtac aagaagagca gagcaggcca 240
gaggacgcac cttcagacct cagtgcccct cactccagtg ccaggctgga cggcacactt 300
gaaggctgtg ctgccgagac gcctggggac tctgcaggtg acatgcgtgt tccagcggac 360
agctccttac tctgtacttt gtcctcagag tctcctcagg aagcagctag tgacgctgag 420
agtggtaaaa agcacgataa caacagcaag tctgcgtccc ggccagacgt tggcaccagc 480
tccccagagc ccactccctt agatcaggaa atgttcaact ccttccattt ctggaggact 540
cctctacccc agatagatct tgataaagag ctccaacagg accctgggga gaggcccagc 600
ccagagagaa caggagatgc acctgcagcc cctgtaccag gttctcccag tatcaccatg 660
gctacccgga aggaactaga agaaatgata gaaaacctag agccgcacat ggatgacccg 720
gatgttaaag cccaggtgga agtgctgtcg gccgccctgc gcgcttctac cctggatgct 780
cacgacgagg ctggcggtgc agagcagcgg agtgagctgc aggacgacgc agtgggtgcc 840
ggcggcgagc ttccaaactg tagcatcagc gaagacactt ctgagcctct ggtcatcgct 900
gctgaggaga atatggaggc cactcctgac tatatccatg gaggtgcgga tgtaggcccc 960
ggtggcggtg gtggcttcag cccggatgaa gagaggagac ccaaagtcca ggatgtcgta 1020
ccacaagcgt tactagacca gtacctgtca atgaccgacc cttctcgagc acagacagtc 1080
gacaccgaga tcgctaagca ctgtgcatac agtctgccgg gtgtggctct ga 1132

## Description

### Technical Field

The present invention belongs to the field of genetic engineering and specifically relates to isolation of a gene of renal cells.

### Background Art

Sixty trillion various cells in *vivo* essentially comprise identical genomic DNA. For the normal physiological functions, the expression of these genes is strictly controlled by signals received by cell lines and cells. Therefore, elucidation of genes expressed in each cell type is very important.

A mesangial cell plays a pivotal role in maintaining the structure and function of a glomerulus and also has a central meaning of pathophysiology for each type of nephritis. For example, proliferation of mesangial cells and accumulation of extracellular mesangial matrix are thought to be important pathological finding of glomerulosclerosis in a patient suffering from various glomerular diseases such as chronic nephritis and diabetic nephropathy. Therefore, identification of genes expressed specifically in mesangial cells and elucidation of its function are helpful for understanding biological characteristics of mesangial cells and the causes of diseases relating to mesangial cells, and in turn, treating or diagnosing diseases relating to mesangial cells.

Thyl antigen is known as a marker for mesangial cells in rats. However, this gene is not specific to mesangial cells and is not expressed in human mesangial cells (Miyata T. et al., Immunology, 1989, 67: 531-533; and Miyata T. et al. , Immunology, 1990, 69:391-395). Mesangial cells are known to express α smooth muscle actin when activated, but this gene is also not specific to mesangial cells. Any genes characteristically in mesangial cells have not been reported.

The present inventor has previously reported MEGSIN as a protein that is expressed specifically in the mesangial cells (J. Clin. Invest, 1998 Aug 15, 120: 4, 828-36). The present invention relates to a novel protein having a structure that is distinctly different from the MEGSIN.

### Disclosure of the Invention

An objective of the present invention is to isolate a gene highly expressed in mesangial cells.

The current inventor isolated mRNA from *in vitro* cultures of human mesangial cells to construct a cDNA library of 3' side. Then, numerous clones in cDNA library were randomly selected, and determined their sequences. Next, determined sequences were compared with the known nucleotide sequences of cDNA clones of 3' side obtained from various organs and cells to determine the clones expressed in mesangial cells. Among the clones, one clone that frequently appears in mesangial cells was selected, and its full-length cDNA (3928 bp) was isolated by 5' RACE method. The whole nucleotide sequence of this full-length cDNA was determined, and thus the present invention was completed. The Kozak translation initiation codon was determined, and then its open reading frame (ORF) was estimated. The protein of this invention having this deduced amino acid sequence was named Meg-1 by the present inventor. The nucleotide sequence of human Meg-1 cDNA and the deduced amino acid sequence for human Meg-1 are shown in SEQ ID NO: 1 and SEQ ID NO: 2, respectively.

An amino acid sequence homology search performed on this amino acid sequence using the SwissProt database confirmed that Meg-1 is a novel protein. Moreover, motif search on the deduced amino acid sequence of Meg-1 was conducted and it revealed a motif that is commonly found in many known protein phosphatases. Since the score of nuclear localization signal is high, it is suggested to be a nuclear protein.

Furthermore, when the topography of Meg-1 was detected by Northern blotting, high expression of Meg-1 was observed in the heart and placenta followed by the expression in the brain, skeletal muscle, kidney, and pancreas. Additionally, weak expression was observed in liver, and expression of Meg-1 was hardly observed in lung. Characteristically, it was highly expressed in mesangial cells in particular, among primary cultured cells. This invention was completed based on these findings.

This invention specifically relates to the following polynucleotide, protein, and their uses.
(1) A polynucleotide selected from the group consisting of (a) to (d) below:
   (a) a polynucleotide comprising a protein-coding region of the nucleotide sequence of SEQ ID NO: 1;
   (b) a polynucleotide encoding a protein comprising the amino acid sequence of SEQ ID NO: 2;
   (c) a polynucleotide comprising the amino acid sequence of SEQ ID NO: 2 in which one or more amino acids are substituted, deleted, inserted and/or added, said polynucleotide encoding a protein functionally equivalent to a protein comprising the amino acid sequence of SEQ ID NO: 2; and
   (d) a polynucleotide hybridizing to a DNA comprising the nucleotide sequence of SEQ ID NO: 1 under stringent conditions, said polynucleotide encoding a protein functionally equivalent to a protein comprising the amino acid sequence of SEQ ID NO: 2.
(2) A protein encoded by the polynucleotide of (1).
(3) A vector comprising the polynucleotide of (1).
(4) A transformant carrying the polynucleotide of (1) or the vector of (3).
(5) A method for producing the protein of (2), said method comprising culturing the transformant of (4) and recovering an expression product.
(6) An oligonucleotide having a chain length of at least 15 nucleotides, said oligonucleotide comprising a nucleotide sequence complementary to the nucleotide sequence of SEQ ID NO: 1 or to a complementary strand thereof.
(7) A method for detecting a polynucleotide comprising the nucleotide sequence of SEQ ID NO: 1, said method comprising contacting the oligonucleotide of (6) with a test sample and observing hybridization of said oligonucleotide.
(8) A method for synthesizing a polynucleotide comprising the nucleotide sequence of SEQ ID NO: 1, said method comprising contacting the oligonucleotide of (6) with a test sample and synthesizing the complementary strand using said oligonucleotide as a primer.
(9) A reagent for detecting mesangial cells, said reagent comprising the oligonucleotide of (6).
(10) A method for detecting mesangial nephritis, said method comprising measuring an expression level of the polynucleotide of (1) in a biological sample, and comparing said level with that obtained from a normal sample.
(11) The method of (10), wherein said biological sample is a mesangial cell.
(12) The method of (10), wherein said expression level is measured using, as an indicator, an mRNA comprising a nucleotide sequence selected from the nucleotide sequence of SEQ ID NO: 1.
(13) The method of (10), wherein said expression level is measured using, as an indicator, the protein of (2) or a fragment thereof.
(14) An antisense polynucleotide against the polynucleotide of (1) or a portion thereof.
(15) An antibody recognizing the protein of (2).
(16) The antibody of (15), recognizing a portion of a protein comprising an amino acid sequence selected from the amino acid sequence of SEQ ID NO: 2.
(17) An immunological assay for measuring the protein of (2) or a fragment thereof based on immunological binding of the antibody of (15) to the protein of (2) or a fragment thereof.
(18) A reagent for immunological assay of the protein of (2) or a fragment thereof, said reagent comprising the antibody of (15).
(19) A transgenic non-human vertebrate, wherein an expression level of a gene encoding Meg-1 is altered.
(20) The transgenic non-human vertebrate of (19), wherein said non-human vertebrate is a mouse.
(21) The transgenic non-human vertebrate of (20), wherein said transgenic non-human vertebrate is a knockout mouse in which the expression of a gene encoding Meg-1 is inhibited.

To fulfill the issues mentioned above, the present inventor used a 3'-directed cDNA library. This method avoids the variation of cloning efficiency affected by size of DNA. The sequence at the 3' region characterizes each of the genes, and the sequence data of approximately 200 to 300 bp are large enough to demonstrate the characteristics of the gene (Yasuda Y., Miyata T. et al., Kidney Int, 1998 Jan, 53:1, 154-8).

The polynucleotide encoding human Meg-1 of the present invention can be obtained by preparing mRNA from mesangial cells and converting them to the double stranded cDNA by the known methods. mRNA can be prepared by, for example, the guanidine isothiocyanate-cesium chloride method (Chirwin, et al., Biochemistry 18, 5294, 1979), and the treatment with a surfactant and phenol in the presence of deoxyribonuclease (Berger & Birkenmeier, Biochemistry 18, 5143, 1979), etc. Preparation of poly (A)⁺ RNA from total RNA can be performed by, for example, the affinity chromatography using such a carrier bound to oligo(dT) as Sepharose, cellulose, latex particles, etc. The cDNA (1st strand) can be obtained by treating the above-mentioned obtained mRNA as the template and the oligo (dT), which is complementary to the poly (A) chain at the 3'-terminus, the random primer or the synthetic oligonucleotide, which corresponds to part of the amino acid sequence of Meg-1, as the primer with a reverse transcriptase. The mRNA in the hybrid composed of mRNA and cDNA complementary thereto is partially cleaved with the *E. coli* RNase H. Using the cleaved mRNA as the primer, cDNA (2nd strand) is synthesized by *E.coli* DNA polymerase I. Finally, the double strand cDNA can be obtained by treating with *E.coli* DNA ligase.

The DNA can be cloned by RT-PCR method using poly (A)⁺ RNA from mesangial cells as a template, and primers synthesized based on the human Meg-1 gene nucleotide sequence. Alternatively, without using PCR, the target cDNA can be obtained by directly screening a cDNA library with a probe synthesized based on human Meg-1 gene nucleotide sequence. The gene of the present invention can be selected by confirming the nucleotide sequence of the gene among the genes obtained by these methods. The Meg-1 cDNA homologues in species other than humans, such as mouse or rat, can be obtained using similar methods.

Otherwise, the Meg-1 cDNA homologues can be isolated as follows. The cDNA encoding the Meg-1 homologue can be isolated by screening the cDNA library by colony hybridization and plaque hybridization, using the nucleotide sequence of the above-mentioned human Meg-1 cDNA as a probe. The cDNA library can be synthesized by using mRNA isolated from mouse or rat tissues, cultured mesangial cells, and the like as template. A commercially available cDNA library (Funakoshi, etc.) may also be used. PCR that utilizes a degenerative primer, which is designed to be placed before and after ORF, based on the cDNA of human Meg-1 of this invention is another possible method that amplifies the cDNA of homologues. A rat homologue of Meg-1 that was obtained based on such a method is disclosed in Examples.

The human Meg-1 genome can be obtained by screening a genomic library. A genomic library can be synthesized, for example, by preparing a genome from human B lymphoblasts and by inserting, into phage vector EMBL3, DNAs partially digested with Sau3 (Blood, vol 83, No 11, 1994: pp 3126-3131). A clone containing the desired genome can be obtained by performing plaque hybridization (see, Shin Saibou Kougaku Jikken (New Cell Biotechnology Experiment) Protocols, Shujun-sha, pp79-92) for such a genomic library. The entire open reading frame region of Meg-1 cDNA (2850 bp), or each of the exon-intron portions obtained by amplifying the human genomic DNA by PCR method using part of the cDNA as primers can be used as probes. A sequence of 5' UTR of the control region sequence can be determined by 5' RACE method (5'-Full RACE Core Set, following Takara's protocol) using human cultured mesangial cell-derived mRNA or human renal mRNA (purchased from Clontech) as a template.

The gene of the present invention can also be produced by following the standard methods using chemical synthesis of nucleic acids, such as phosphoamidite method (Mattencci, M. D. & Caruthers, M. H. J. Am. Chem. Soc. 103, 3185, 1981), phosphite triester method (Hunkapiller, M. et al., Nature 310, 105, 1984).

An eukaryotic gene often shows polymorphism, like human interferon gene, and one or more amino acids may be replaced by this polymorphism with generally maintaining activities of a protein. In general, activities of proteins can be often maintained even if one or more amino acids are modified. Therefore, polynucleotide encoding a protein obtained by using the artificially modified gene encoding an amino acid sequence of SEQ ID NO: 2 is included in this invention as long as the protein possesses the function typical to the gene of the present invention. The present invention includes protein in which an amino acid sequence of SEQ ID NO: 2 is artificially modified as long as it has characteristics of the proteins of the present invention.

The proteins of the present invention comprise the amino acid sequence of SEQ ID NO: 2 or the amino acid sequence in which one or more amino acids are replaced, deleted, added, and/or inserted, and being functionally equivalent to Meg-1 protein of present invention. In this invention, "functionally equivalent" means having biological properties that are the same as Meg-1. The inventor has found the following biological properties in Meg-1, for example.

First of all, Meg-1 shows, in its amino acid sequence, 20 to 30% homology to protein phosphatase 2A (PP2A) subunit A (regulatory subunit) from a variety of species including human. PP2A is known to form a trimer in which subunit C (a catalytic subunit having enzyme activity) is bound to the C terminal region of subunit A and subunit B (another regulatory subunit specific for tissues and cells) to the N terminal region of subunit A. While subunits A and C have. a common structure among the tissues and cells, subunit B is thought to have a structure specific for tissues and cells. Based on this information, subunit B having, as subunit A, Meg-1 of the present invention that shows homology to PP2A has the possibility of a mesangial cell-specific protein. Subunit B having Meg-1 as subunit A is thought to be involved in a mechanism for regulating the mesangial cell-specific activity.

The characteristic feature of the Meg-1 expression was a particularly high level of expression in the primary culture of mesangial cells. In other primary cell cultures, its expression was observed in dermal fibroblasts and renal cortical epithelial cells, and also in endothelial cells of umbilical vein and smooth muscle cells at trace levels. When the expression in the different tissues were compared, abundant expression of Meg-1 was observed in heart and placenta, and then in brain, skeletal muscle, kidney and pancreas. In addition, low-level expression of the gene was observed in liver, but no expression in lung. Among the tumor cell lines, high level expression of the gene was observed in HeLa cell line S3, chronic myelocytic leukemic cell line K-562, lymphoblastic leukemia cell line MOLT-4, colon adenocarcinoma cell line SW480 and lung carcinoma cell line A549. Also, promyelocytic leukemia cell line HL-60 and melanoma cell line G361 were observed to express the gene. Expression in Burkitt's lymphoma Raji was almost negligible. Analysis of the rat Meg-1 expression in the rats showed that Meg-1 was highly expressed in the kidney (particularly in mesangial cells) both in humans and rats, suggesting the possibility that Meg-1 is one of the functional genes responsible for mesangial cell function. The state of Meg-1 expression in each tissue and cultured cell can be investigated by Northern blotting assay using mRNA prepared from each tissue as a sample, with a probe having, for example, a sequence selected from the nucleotide sequence of SEQ ID NO: 1.

Any proteins functionally equivalent to Meg-1 for the biological features as described above will constitute Meg-1 of the present invention. Thus, the present invention includes not only human Meg-1, whose structure has been specifically elucidated, but also its homologues derived from other species, which have equivalent structure and functions.

Among the known amino acid sequences verified by homology search, PP4_{R1} (J.Biol.Chem.274(9), 5339-5347, 1999) shows the closest structural similarity with Meg-1. However, PP4_{R1} is a protein that has been reported to be a human homologue of a regulatory subunit of protein serine/threonine phosphatase 4 isolated from the bovine small intestine. In their structures, the serine residue located at position 18 from the N-terminus of PP4_{R1} is replaced with 18-amino acid residues of FGVDDYSSESDVIIIPSA in Meg-1. Thus, the two proteins are different in the structure. Also, from a functional point of view, they are different, because PP4_{R1} was isolated from neuroepithelial cells, based on the amino acid sequence of a protein from bovine small intestine, but in contrast, Meg-1 is strongly expressed in mesangial cells. Since the two proteins have continuous amino acid sequences different from each other, it is possible that both Meg-1 and PP4_{R1} are isoforms encoded by a plurality of genes or that they are splicing variants.

The polynucleotide of the present invention includes polynucleotides encoding these functionally equivalent proteins. The polynucleotides encoding these proteins can be cDNA, genomic DNA, synthetic DNA, RNA, and artificial polynucleotide molecule constituted by nucleotide derivatives.

The codons for desired amino acids themselves are well-known, can be optionally selected, and can be determined by following the standard method by, for example, considering the frequency of use of codons in hosts to be used (Grantham, R. et al. Nucleic Acids Res. 9, r43, 1981). Therefore, the present invention includes polynucleotides appropriately modified by degeneration of codons. It is possible to partially change the codons of these nucleotide sequences by following site-specific mutagenesis methods (Mark, D. F. et al., Proc. Natl. Acad. Sci. U.S.A. 1984, 81: 5662) and such that uses chemically synthesized oligonucleotides encoding the desired modifications as primers.

A polynucleotide hybridizing with a polynucleotide containing the nucleotide sequence of SEQ ID NO: 1 and encoding a protein that typically functions as Meg-1 of the present invention, can be included in the polynucleotides of the present invention. A sequence capable of hybridizing with specific sequences under stringent conditions is thought to have the activities similar to a protein encoded by the specific sequences. Stringent conditions generally indicate the following conditions. That is, hybridization is carried out at 65°C in 4X SSC, and then washing is carried out for one hour at 65°C using 0.1X SSC. The temperature of hybridization and washing, which greatly affect stringency, can be adjusted depending on the melting temperature (Tm). Tm changes, depending on the proportion of constituent bases occupying the base pairs to be hybridized, and by the composition of the hybridization solution (salt concentration, concentrations of formamide and sodium dodecyl sulfate) . Therefore, through experience, one skilled in the art can consider these conditions mentioned above to set appropriate conditions that will yield similar stringency.

The nucleotide sequences of polynucleotides of the present invention, including mutants, can be used for various purposes based on known techniques.

Other prokaryotic or eukaryotic hosts can be transformed by inserting the polynucleotide encoding Meg-1 cloned as described above into an appropriate expression vector DNA. Moreover, the gene can be expressed in each host cell by introducing an appropriate promoter and sequences relating to the phenotypic expression into the expression vector. As an expression vector, for example, pET-3 (Studier & Moffatt, J. Mol. Biol. 189, 113, 1986) and such for E. *coli,* pEF-BOS (Nucleic Acids Research 18, 5322, 1990), pSV2-gpt (Mulligan & Berg, Proc. Natl. Acad. Sci. U. S. A. 78, 2072, 1981), and so on for COS cells, and pVY1 (WO89/03874) and such for CHO cells can be used. The target proteins can be expressed as a fusion protein derived from a fusion gene between a target gene and a gene encoding other polypeptide. Such fusion proteins can easily be purified and separated to isolate a desired protein. Histidine tag, c-myc tag, MBP-tag, GST-tag, and the like are known as proteins to be fused. Vectors that can express inserts in which these tags are fused are commercially available.

For example, *Escherichia coli* can be used as prokaryotic host cells in the expression system of the present invention. *Saccharomyces cerevisiae* and such can be used as microbiological host cells among eukaryotic organisms. Examples of mammalian host cells include COS cells, CHO cells, BHK cells, etc. The transformants of the current invention can be cultured under appropriately selected culturing condition suitable for host cells.

As mentioned above, Meg-1 can be produced by culturing the transformants transformed with the polynucleotide encoding the target Meg-1, and recovering it from the cells or the culture supernatant. It can be purified into a substantially pure protein. Meg-1, a target protein of the present invention, can be separated and purified by the separation and purification methods commonly used for proteins, and the method is not particularly limited. Meg-1 can be separated and purified by, for example, appropriately selecting and combining various chromatographies.

Besides the methods descried above, the gene of the present invention, the recombinant vector comprising the gene, the transformants carrying the vector, and the production of Meg-1 using gene manipulation can be manipulated by the standard method described in "Molecular Cloning - A Laboratory Manual" (Cold Spring Harbor Laboratory, N. Y.).

In addition, a probe for detecting a Meg-1 gene can be designed based on the nucleotide sequence of SEQ ID NO: 1. Moreover, primers for amplifying DNA and RNA containing these nucleotide sequences can be designed. It is routine for a person skilled in the art to design probes and primers based on a given sequence. An oligonucleotide comprising a designed nucleotide sequence can be chemically synthesized. These oligonucleotides can be used for the hybridization assay of various formats, or for the synthetic reaction of nucleic acids, such as PCR, if appropriately labeled. An oligonucleotide used as a probe or a primer has at least 15 bases, and preferably 25 to 50 bases. Probes (or primers) that can distinguish Meg-1 and PP4_{R1} can be prepared by designing them to contain a nucleotide sequence selected from a part corresponding to the 18 amino acid-encoding region (74-127) that is structurally different to PP4_{R1}, which shares almost same nucleotide sequence.

The present invention provides a polynucleotide comprising at least 15 nucleotides and complementary to a polynucleotide comprising the nucleotide sequence of SEQ ID NO: 1 or its complementary strand. Herein, the term "complementary strand" is defined as one strand of a double strand polynucleotide composed of A:T (in RNA, T is replaced to read U) and G:C base pair to the other strand. Also, "complementary" is defined as not only those completely matching within a continuous region of at least 15 nucleotides, but also having a homology of at least 70%, favorably 80%, more favorably 90%, and most favorably 95% or higher within that region. The homology may be determined using the. algorithm described herein. Since Meg-1 is highly expressed in mesangial cells, probes and primers for detecting the Meg-1 gene of this invention can be used to identify and detect the mesangial cells. Primers are especially useful to amplify the Meg-1 gene. If necessary, labels or binding tags can be added to the oligonucleotides of the invention.

The present invention also provides a method for detecting mesangial nephritis using Meg-1 expression as an indicator. Expression of the polynucleotide of the present invention is increased in the mesangial cells of the kidney tissue in which mesangial nephritis has been developed. This is supported by the observation obtained in the working example that rat Meg-1 mRNA was strongly expressed in the rat mesangial cells in which mesangial nephritis was induced by administration of anti-Thyl monoclonal antibody. Therefore, mesangial nephritis can be detected when Meg-1 expression is increased, by measuring Meg-1 expression in a biological sample and comparing the measurement with that obtained from a normal sample. Mesangial cells, urine and blood may be used as the biological samples. Meg-1 expression levels may be compared by measuring mRNA or its translational product Meg-1 protein, or fragments thereof. Methods for measuring these components in biological samples are well known. For example, Meg-1 mRNA may be detected or measured by Northern blotting or RT-PCR. Meg-1 protein or a fragment thereof may be detected using an antibody recognizing Meg-1. Where mesangial cells are used as a biological sample, cellular localization of these components can be elucidated using *in situ* hybridization or immunohistological detection.

Furthermore, an antisense polynucleotide that may regulate the expression of Meg-1 is provided based on the nucleotide sequence of the gene encoding Meg-1 disclosed in this invention. The antisense polynucleotide of this invention is an important tool for demonstrating the role of Meg-1 in the mesangial cells. It is also useful for regulating diseased conditions caused by accelerated expression of Meg-1. The effects of inhibiting the expression of target genes by antisense polynucleotides are the following. They are inhibition of transcription initiation by triple strand formation, transcription inhibition by hybrid formation with a site that has formed a local open loop structure due to RNA polymerase, transcription inhibition by hybrid formation with RNA that is being synthesized, splicing inhibition by hybrid formation at the joint between intron and exon, splicing inhibition by hybrid formation with the spliceosome forming region, inhibition of mRNA transition from the nucleus to the cytoplasm by hybrid formation with mRNA, splicing inhibition by hybrid formation with the capping region and the poly (A) attached region, inhibition of translation initiation by hybrid formation with the translation initiation factor binding region, translation inhibition by hybrid formation with the ribosome binding region near the initiation codon, interception of protein chain elongation by hybrid formation with the translation region of mRNA and polysome binding region, and inhibition of gene expression by hybrid formation with the nucleic acid-protein interacting region, and so on. These inhibit the expression of the target gene by inhibiting the transcription, splicing, or translation processes (Hirashima and Inoue, Shin Seikagaku Jikken Koza 2 Kakusan IV Idenshi no Fukusei to Hatsugen (New Biochemistry Laboratory Experiment 2 Nucleic Acids IV Replication and Expression of Genes), Japanese Biochemical Society Edition, Tokyo Kagaku Dojin, pp. 319-347, 1993).

The antisense sequence used in this invention may inhibit the expression of the target genes by any one of the effects mentioned above. In one embodiment, if an antisense sequence complementary to the non-translational region located near the 5' end of mRNA of the gene is designed, it may be effective for inhibiting the translation of the gene. However, sequences complementary to the coding region or to the 3'end non-translational region may also be used. In this manner, the antisense polynucleotide utilized in this invention includes polynucleotide containing the antisense sequence of not only the translational region of the gene, but also the sequence of the non-translational region. The antisense polynucleotide to be utilized is inserted downstream of the appropriate promoter, and preferably, a sequence including a transcription-terminating signal is inserted to the 3' end. DNA prepared in this manner can be transformed into the desired host by known methods. Preferably, the antisense polynucleotide sequence should contain a sequence that is complementary to the endogenous gene (or its homologous genes) of the host to be transformed or its portion. However, as long as gene expression is inhibited effectively, complete complementarity is not necessary.

The RNA that is transcribed using antisense polynucleotide as template is designed to have preferably 90% and most preferably 95% complementarity to the transcription product of the target gene. The length of the antisense polynucleotide used for effectively inhibiting the expression of target genes is at least 15 nucleotides or more, preferably 100 nucleotides or more, and more preferably 500 nucleotides or more. Usually, the length of the antisense RNA used is shorter than 2.5 kb.

A promoter region and an enhancer region of Meg-1 gene existing in genome can be obtained based on the cDNA nucleotide sequence of Meg-1 of the present invention. Specifically, these control regions can be obtained by the same method as described in unexamined published Japanese patent application (JP-A) No. Hei 6-181767; The Journal of Immunology, 1995, 155, 2477-2486; Proc. Natl. Acad. Sci. USA, 1995, 92, 3561-3565; etc. Herein, a promoter region means DNA region existing upstream of a transcription initiation site to control the expression of a gene, and an enhancer region means DNA region existing in an intron or 3'UTR to control expression of a gene.

Specifically, a promoter region can be obtained, for example, by the following method.
1) A promoter region of Meg-1 is cloned from a human genomic library using 5' end site of cDNA of Meg-1 as a probe.
2) Meg-1 gene is digested with restriction enzyme to obtain a DNA comprising the promoter region at the upstream region (2 to 5 kbp) containing a translation initiation codon of Meg-1 gene and determine the nucleotide sequence. The transcription initiation site (+1) is determined using poly(A)⁺RNA prepared from human mesangial cells as a template, by the primer elongation method using primer DNA selected from cDNA sequence at 5' end site of Meg-1 gene. A site possibly comprising the promoter activity is predicted by searching transcription factor binding sequence from the nucleotide sequence.
3) The DNA fragment excluding the coding region of Meg-1 gene from the DNA obtained in 2) is subcloned in a plasmid, and a chloramphenicol acetyl transferase (CAT) gene or a luciferase gene is ligated as a reporter gene at 2 to 5 kbp downstream of the DNA fragment to construct a reporter plasmid. Similarly, DNA fragments corresponding to various sites upstream of Meg-1 gene, in which 5' and 3' end sites are stepwise removed, are prepared by digestion with restriction enzymes or by PCR to include possible promoter regions. The CAT gene or the luciferase gene is ligated as a reporter gene at downstream of these DNA fragments to construct a reporter plasmid.
4) A promoter region upstream of Meg-1 gene is obtained by measuring CAT or luciferase activity in animal cells transformed with the reporter plasmid prepared in 3).

A 3' UTR and an enhancer region in introns can be obtained by cloning genomic genes of human Meg-1 from a human genomic library using Meg-1 cDNA as a probe in the same manner as described above for the promoter.

Transcription factors controlling the expression of Meg-1 gene can be obtained by the known methods, for example, those described in "Shin Saibou Kougaku Jikken (New Cell Biotechnology Experiment) Protocols, Shujun-sha," "Biomanual series 5 Tensha Inshi Kenkyu-hou (studies on transcription factors), Yodo-sha," "DNA & Cell Biology, 13, 731-742, 1994," such as affinity chromatography, South-western method, footprinting method, gel shift method, or one-hybrid method. Herein, a transcription factor means a factor controlling the transcription of Meg-1 gene, including a transcription initiation factor that induces the transcription initiation reaction and a transcription control factor that up- or down-regulates transcription.

Affinity chromatography can be performed by applying a nucleic extract to an affinity column in which promoter and enhancer regions obtained above are immobilized on Sepharose or latex beads, washing the column, eluting the binding transcription factor using a DNA comprising the same sequence as that immobilized in the column, and recovering the transcription factor controlling the expression of Meg-1 gene.

In case where the South-Western method is employed, the expression product of the cDNA inserted in the *E.coli* expression vector (e.g. λgt11) is screened as per the tagged-probe. For example, the cDNA to be screened is expressed as a fusion protein with the β-galactosidase, and is adsorbed to the nitrocellulose filter. Then, the transcription factor, which regulates the Meg-1 gene expression, can be obtained by selecting those phages that synthesize the fusion protein with a binding activity using radioisotope tagged DNA fragments of the promoter region or enhancer region as the probe.

The gel shift method is based on the phenomenon that electrophoretic mobility on a polyacrylamide gel of DNA changes when it is bound to a protein. DNA fragments of the promoter region and enhancer region are used as probes and upon mixing with samples containing transcription factors (for example, nuclear protein extract), they are analyzed by electrophoresis under low ionic strength. The binding of transcription factors is detected as a band with mobility different from that of free DNA. The gel shift method allows separation of transcription factors from a mixture of proteins with high sensitivity.

Upon further analysis by the footprint method, the DNA-transcription factor complex obtained by the gel shift method allows determination of the transcription factor-binding site. The footprint method utilizes the phenomenon that when a protein binds to DNA, it is protected from DNase I digestion. That is, DNA of the promoter region and enhancer region labeled at the end with ³²p is partially digested by DNase I in the presence of transcription factors, and then separated by degenerate polyacrylamide gel used for determining nucleotide sequences. Comparison to the result when the same treatment is carried out in the absence of a transcription factor shows the disappearance of a band due to transcription factor binding, and therefore, estimation of the binding region becomes possible.

The present invention also provides an antibody recognizing Meg-1. The antibody of the present invention includes, for example, an antibody against the protein comprising the amino acid sequence of SEQ ID NO: 2. An antibody (for example, a polyclonal antibody, a monoclonal antibody) or an antiserum against Meg-1 or a partial peptide of Meg-1 of the present invention can be produced by a known method for producing an antibody and antiserum, using Meg-1 of the present invention, a partial peptide of Meg-1 of the present invention, or a fusion protein such as c-myc-(His)₆-Tag-Meg-1 or MBP-Meg-1 of the present invention as a antigen.

The Meg-1 of the present invention or a partial peptide of Meg-1 of the present invention is administered with well-known carrier or diluent to a warm-blooded animal at the site capable of producing an antibody. To enhance the antibody productivity, the complete Freund's adjuvant or incomplete Freund's adjuvant can be administered together with the antigen. Immunization is performed every one to six weeks, a total of about 2 to 10 times, in general. Warm-blooded animals to be used are, for example, a monkey, rabbit, dog, guinea pig, mouse, rat, sheep, goat, and chicken, and preferably a mouse and rabbit.

Monoclonal antibody-producing cells can be prepared by selecting immunized warm-blooded animals, such as mice, in which an antibody titer is detected, obtaining spleen or lymph node from the animals 2 to 5 days after the final immunization, and fusing the antibody producing cells contained in these tissues with myeloma cells to obtain monoclonal antibody-producing hybridoma. The antibody titer in antiserum can be measured by, for example, reacting the labeled Meg-1 described below with antiserum, and measuring an activity of the label binding to the antibody. Cell fusion can be performed by a known method, for example, the method of Kohler and Milstein (Nature, 256, 495, 1975). Polyethylene glycol (PEG), Sendai virus, and such can be used as a fusion enhancer, and PEG is preferable.

Examples of myeloma cells include X-63Ag8, NS-1, P3U1, SP2/0, AP-1, and such, and X-63Ag8 is preferably used. The ratio of the number of antibody-producing cells (splenic cells) to that of myeloma cells is 1:20 to 20:1. Cells can be fused efficiently by adding PEG (preferably PEG1000 to PEG6000) at the concentration of about 10 to 80%, and incubating for 1 to 10 min at 20 to 40°C, preferably at 30 to 37°C. Anti-Meg-1 antibody-producing hybridoma can be screened by various methods, for example, the method in which the hybridoma culture supernatant is added to a solid phase (for example, a microplate) on which Meg-1 antigen is adsorbed directly or with a carrier, and anti-immunoglobulin antibody labeled with a radioactive substance, an enzyme, and such (When cells used for cell fusion are derived from a mouse, anti-mouse immunoglobulin antibody is used.) or protein A is added thereto, and anti-Meg-1 monoclonal antibody binding to the solid phase is detected, the method in which the hybridoma culture supernatant is added to a solid phase on which anti-immunoglobulin antibody or protein A is adsorbed, and Meg-1 labeled with a radioactive substance, an enzyme, and such is added thereto, and anti-Meg-1 monoclonal antibody binding to the solid phase is detected.

Anti-Meg-1 monoclonal antibody can be selected and cloned by known methods or modified methods thereof using usually a culture medium for animal cells supplemented with HAT (hypoxanthine, aminopterin, and thymidine). Any medium for selection, cloning, and culturing can be used as long as hybridoma can grow therein. For example, RPMI 1640 medium (Dainippon Pharmaceutical) containing 1 to 20%, preferably 10 to 20% of fetal bovine serum, GIT medium (Wako Pure Chemicals) containing 1 to 10% fetal bovine serum, or serum-free medium for hybridoma culturing (SFM-101, Nissui Pharmaceutical) can be used. Incubation temperature is generally 20 to 40°C, preferably about 37°C. Incubation time is generally 5 days to 3 weeks and preferably 1 to 2 weeks. Incubation is performed under the 5% carbon dioxide gas in general. The antibody titer of the hybridoma culture supernatant can be determined in the same manner as described above for the measurement of anti-Meg-1 antibody titer in the antiserum. Cloning can be generally conducted by known methods, for example, semisolid agar method, or limiting dilution method. A cloned hybridoma is cultured preferably in a serum-free medium, thereby producing an optimal amount of an antibody in the supernatant. A target monoclonal antibody can be obtained in ascites.

A monoclonal antibody of the present invention does not crossreact with other proteins other than Meg-1 by selecting those capable of recognizing epitopes specific to Meg-1. In general, an epitope specific to a protein is composed of at least 7 or more continuous amino acid residues, preferably 10 to 20 amino acids in an amino acid sequence of the protein. Therefore, a monoclonal antibody recognizing an epitope composed of peptides having an amino acid sequence selected from the amino acid sequence of SEQ ID NO: 2 and composed of at least 7 continuous amino acid residues can be the monoclonal antibody specific to Meg-1 of the present invention. More specifically, for example, it can be said that an antibody recognizing the 18 amino acid-comprising region (18 to 35^{th} residues from the N-terminus) that is structurally different to PP4_{R1}, and distinguishing structural differences with a highly homologous protein such as PP4_{R1}, is a preferable embodiment of the antibody recognizing Meg-1 of this invention.

An anti-Meg-1 monoclonal antibody can be separated and purified by the separation and purification method of immunoglobulin commonly used for the separation and purification of polyclonal antibodies. The known purification methods include, for example, salting out, alcohol precipitation, isoelectric point precipitation, electrophoresis, adsorption and desorption method by ion exchanger (for example, DEAE), ultra centrifugation, gel filtration, or specific purification method whereby antibody is exclusively collected by, for example, an antigen binding solid phase or active adsorbent, such as Protein A or Protein G, and the binding is dissociated to obtain the antibody.

Monoclonal antibodies and polyclonal antibodies recognizing Meg-1 of the present invention, obtained in such a manner, can be used for the diagnosis and treatment for diseases relating to mesangial cells. These antibodies can be used as a tool for identifying or detecting mesangial cells because Meg-1 is a protein that is highly expressed in mesangial cells. Methods for immunologically detecting cellular proteins are commonly known. Examples of a method for measuring Meg-1 with these antibodies include an sandwich assay comprising reacting Meg-1 with an antibody binding to an insoluble carrier and a labeled antibody and detecting Meg-1 in the sandwiched complex produced by the reaction, or a competition method comprising competitively reacting labeled human Meg-1 and human Meg-1 in a sample with an antibody to measure human Meg-1 in a samples based on labeled antigen amount reacted with the antibody.

The measurement of human Meg-1 by the sandwich method is conducted by, for example, the 2 step method in which an immobilized antibody is reacted with Meg-1, unreacted materials are completely removed by washing, and a labeled antibody is added to form a complex of the immobilized antibody Meg-1 the labeled antibody, or one step method in which the immobilized antibody, the labeled antibody, and Meg-1 are mixed at the same time.

Examples of an insoluble carrier used for the measurement include, for example, polystyrene, polyethylene, polypropylene, polyvinyl chloride, polyester, polyacrylate, nylon, polyacetal, synthetic resin such as fluoride resin, etc., polysaccharides such as cellulose, agarose, and such, glass, metals, etc. The form of an insoluble carrier can be varied and includes tray, spheroid, particle, fiber, stick, board, container, cell, test tube, etc. The antibody-adsorbed carrier should be stored at a cool place in the presence of appropriate preservatives, such as sodium azide.

Antibodies can be immobilized by known chemical binding or physical adsorption methods. Chemical binding methods include, for example, a method using glutaraldehyde, the maleimide method using N-succinimidyl-4-(N-maleimidomethyl)cyclohexane-1-carboxylate, N-succinimidyl-2-maleimidoacetate, and such, and the carbodiimide method using 1-ethyl-3-(3-dimethylaminopropyl)carbodiimide hydrochloride, etc. In addition, the maleimidobenzoyl-N-hydroxysuccinimidoester method, the N-succimidyl-3-(2-pyridyldithio)propionate method, the bisdiazolated benzidine method, and dipalmityllysine method. Alternatively, the complex produced by reacting two different antibodies against a substance to be detected and an epitope is captured with the third antibody immobilized by the above method.

Any label useful for immunoassay can be used without being limited. Specifically, enzymes, fluorescent substances, luminescent substances, radioactive substances, metal chelates, and such, can be used. Preferable labeling enzymes are, for example, peroxidase, alkaline phosphatase, β-D-galactosidase, malate dehydrogenase, *Staphylococcus* nuclease, delta-5-steroid isomerase, α-glycerol phosphate dehydrogenase, triosephosphate isomerase, horseradish peroxidase, asparaginase, glucose oxidase, ribonuclease, urease, catalase, glucose-6-phosphate dehydrogenase, glucoamylase, and acetylcholine esterase, etc. Preferable fluorescent substances include, for example, fluorescein isothiocyanate, phycobiliprotein, rhodamine, phycoerythrin, phycocyanin, allophycocyanin, and orthophthalaldehyde. Preferable luminescent substances include, for example, isoluminol, lucigenin, luminol, aromatic acridiniumester, imidazole, acridinium salt and its modified ester, luciferin, luciferase, and aequorine. Preferable radioactive substances include, for example, ¹²⁵I, ¹²⁷I**,** ¹³¹I**,** 14C, ³H, ³²P**,** ³⁵S**,** etc.

The method for binding the above labels is known. Specifically, direct and indirect labeling can be used. The common direct labeling is the method in which an antibody or an antibody fragment is chemically covalent-bound with a label using a crosslinking agent. Crosslinking agents include N,N'-orthophenylenedimaleimide, 4-(N-maleimidomethyl) cyclohexanoate N-succinimide ester, 6-maleimidohexanoate N-succinimide ester, 4,4'-dithiopyridine, and other known crosslinking agents. The crosslinking agent can be reacted with enzymes and antibodies by the known methods depending on the characteristics of the crosslinking agent. An example of the indirect labeling method comprises binding an antibody to a low molecular weight hapten such as biotin, dinitrophenyl, pyridoxal, or fluorescamine, and indirectly labeling the antibody with the binding partner to the hapten. Avidin and streptoavidin can be used as a recognition ligand for biotin, whereas dinitrophenyl, pyridoxal, or fluorescamine are labeled with antibodies recognizing these haptens. Horseradish peroxidase can be used as an enzyme for labeling antibodies. This enzyme is useful because it can react with many substrates and be easily bound to antibodies by the periodate method. Occasionally, as an antibody, their fragments, for example, Fab', Fab, F(ab')₂ are used. Both polyclonal and monoclonal antibodies can be labeled with an enzyme by the same method. Enzyme-labeled antibodies obtained using the above crosslinking agent can be purified by the known methods such as affinity chromatography, and such, to serve in a more sensitive immunoassay system. Purified enzyme-labeled antibodies are stored with a preservative such as thimerosal and a stabilizer such as glycerol. Labeled antibodies can be lyophilized and stored in the cool and dark place for a long time.

When a label is an enzyme, its substrate and, if necessary, a coloring agent is used for measuring its activity. When peroxidase is used as an enzyme, H₂O₂ is used as a substrate solution and 2,2'-azino-di-[3-ethylbenzothiazolinesulfonic acid] ammonium salt (ABTS), 5-aminosalicylic acid, orthtophenylenediamine, 4-aminoantipyrine, or 3,3',5,5'-tetramethylbenzidine, and such, is used as a coloring agent. When alkaline phosphatase is used as an enzyme, orthonitrophenylphosphate, paranitrophenylphosphate, and such, can be used as substrates. When β-D-galactosidase is used as an enzyme, fluorescein-di-(β-D-galactopyranoside), 4-methylumbelliferyl-β-D-galactopyranoside, and such, can be used as substrates. The present invention also includes an immunoassay reagent for Meg-1, comprising labeled or immobilized monoclonal or polyclonal antibodies, and further includes a kit comprising this reagent and an indicator for detection label and a control sample, etc.

Any biological samples such as body fluid such as blood plasma, serum, blood, urine, tissue fluid, or cerebrospinal fluid and such can be used as samples for measuring the Meg-1 of the present invention as long as they contain Meg-1 or its precursor or a fragment.

In addition, the present invention relates to a transgenic nonhuman vertebrate in which the expression level of Meg-1 gene is altered. Herein, Meg-1 gene includes cDNA, genomic DNA, or synthetic DNA encoding Meg-1. Gene expression includes both steps of transcription and translation. Transgenic animals of the present invention are useful for investigating function and expression control of Meg-1, clarifying mechanisms of development of diseases relating to human mesangial cells, and developing disease model animals used for screening and testing safety of pharmaceuticals.

In the present invention, Meg-1 gene can be modified so as to artificially increase or decrease its expression level compared with the original gene by introducing mutation such as deletion, substitution, insertion, and such, in a part of some important sites (enhancer, promoter, intron, etc.) which control the normal expression of Meg-1 gene. Such modification alters transcription of Meg-1 gene. On the other hand, translation to proteins can be modified by deleting a part of an exon, or replacing a certain codon with a stop codon by introducing point mutation into coding regions. Such mutation can be introduced by the known methods for obtaining transgenic animals.

Transgenic animals means, in a narrow sense, animals into reproductive cells of which an exogenous gene is artificially introduced by genetic recombination, and in a broad sense, animals into chromosome of which an exogenous gene is stably introduced during an early developmental stage, the gene can be transmitted to the offspring as genotype, including antisense transgenic animals in which the function of a specific gene is inhibited by antisense RNA, animals in which a specific gene is knocked out by using embryonic stem cells (ES cells), and animals into which point mutation DNA is introduced. Transgenic animals used herein include all vertebrates except for human.

Transgenic animals can be prepared by the method comprising mixing a gene with an egg and treating the mixture with calcium phosphate, the microinjection method whereby a gene is directly injected into a nucleus in pronuclear egg by a micropipette under the phase contrast microscope (microinjection method, U.S. Patent No. 4,873,191), and the method using embryo stem cells (ES cells). Other methods include, for example, the method in which a gene is inserted into a retrovirus vector to infect an egg and the sperm vector method in which a gene is introduced into an egg through sperm, etc. The sperm vector method is a genetic recombination method for introducing an exogenous gene by attaching an exogenous gene into sperm or incorporating an exogenous gene into sperm cells by electroporation, etc. and fertilizing an egg (M. Lavitranoet et al., Cell, 57, 717, 1989).

*In vivo* site-specific genetic recombination such as cre/loxP recombinase system of bacteriophage P1, FLP recombinase system of *Saccharomyces cerevisiae,* and such, can be used. The method for introducing a transgene of a target protein into nonhuman animals using retrovirus has been reported..

Methods for preparing transgenic animals by microinjection are performed, for example, in the following manner. A transgene basically composed of a promoter regulating expression, a gene encoding a specific protein, and poly (A) signal is provided. Expression pattern and level for all lineages should be confirmed since the expression pattern and level of a specific molecule depend on the promoter activity, and prepared transgenic animals vary among lineages depending on the number of copies and introduction site on chromosomes of an introduced transgene. A sequence of introns to be spliced at upstream of poly (A) signal may be introduced when the expression level is known to vary depending on noncoding region and splicing. It is important to use a gene as pure as possible for introducing into a fertilized egg. An animal to be used includes a mouse for collecting fertilized eggs (5 to 6 weeks old), male mouse for crossing, pseudopregnant female mouse, vasoligated male mouse, etc.

To efficiently obtain fertilized eggs, ovulation can be induced by gonadotropin, etc. A fertilized egg is collected, and a gene is injected into a male pronucleus of the egg by microinjection using an injection pipette. Animals for returning the treated eggs into an oviduct are prepared (pseudopregnant female mice, etc.) , and about 10 to 15 eggs are transplanted per each individual. Introduction of the transgene into a new-born mouse is confirmed by extracting genomic DNA from the tip of the tail and detecting the transgene by Southern hybridization or PCR methods, or by the positive cloning method in which a marker gene that is activated only upon homologous recombination is inserted. Expression of the transgene can be confirmed by detecting a transgene-derived transcript by Northern hybridization or RT-PCR methods. Detection by Western blotting method is also possible using an antibody specific to a protein.

A knockout mouse of the present invention is prepared so as to lose the function of mouse Meg-1 gene. A knockout mouse means a transgenic mouse in which a certain gene is destroyed by homologous recombination technology to eliminate its function. A knockout mouse can be prepared by conducting homologous recombination using ES cells and selecting ES cells in which one allele is modified and destroyed. For example, genetically manipulated ES cells are injected into a blastocyst or an 8-cell embryo of a fertilized egg to obtain a chimeric mouse having both cells derived from ES cells and from embryo. A heterozygous mouse in which all of one allele is modified and destroyed can be prepared by crossing a chimeric mouse (chimera means an individual composed of somatic cells derived from two or more fertilized eggs) and a normal mouse. Crossing of heterozygous. mice with each other can produce homozygous mice. A transgenic animal of the present invention includes both heterozygotes and homozygotes.

Homologous recombination means the recombination occurring between two genes whose nucleotide sequences are the same or extremely similar through mechanism of genetic recombination. Cells with homologous recombination can be selected by PCR. Homologous recombination can be confirmed by performing PCR using as primers sequences of a part of a gene to be inserted and a part of a chromosomal region into which the gene is expectedly inserted and detecting cells producing amplified products. Homologous recombination in the genes expressed in ES cells can be easily screened by known methods or their modified methods, for example, by binding neomycin resistant gene to the introduced gene to make the cells neomycin resistant after the introduction.

### Brief Description of the Drawings

Figure 1 is a micrograph (magnification 40) showing the result of the *in situ* hybridization with Meg-1 specific probe in the normal human glomerulus tissue.
Figure 2 is a micrograph showing the result of the *in situ* hybridization with Meg-1 specific probe in the normal human glomerulus tissue at the magnification higher than that of Figure 1 (magnification 80).
Figure 3 is a photograph showing the result of the electrophoresis of total mRNA prepared from rat glomerulus in which mesangial nephritis was induced by anti-Thyl antibody (represented by "total RNA" in the figure), and a photograph showing the result of Northern blot analysis using Meg-1 specific probe (represented by "rat Meg-1" in the figure).

### Best Mode for Carrying out the Invention

The present invention is illustrated in detail below with references to examples, but is not to be construed as being limited thereto.

### Example 1: Primary culture of human mesangial cells

Human glomerular renal mesangial cells were isolated from the normal human kidney excised from a 58 year-old male. Renal cortex was separated under the sterilized condition, minced, and passed through several sieves. Pore diameters of the used sieves were decreased stepwise, and the trapped glomerulus by the sieve at the pore diameter of 75 to 200 µm was washed and incubated with 100 µg/ml collagenase (Washington Biochemical) at 37°C for 20 min. After washing, the glomerulus was resuspended in medium 199 (Gibco BRL, Gaithersburg, MD) containing 25 mM Hepes, 10% Nu-serum (Collaborative Biomedical Products, Bedford, MA), and antibiotics (10 µg/ml of penicillin, streptomycin, and fungizone), and incubated in the 5% CO₂ incubator. At the third passage, mesangial cells were identified based on a series of criteria such as typical morphological characteristics, resistance to trypsin, puromycin, and D-valine, positiveness against immunostaining of actin (Zymed Laboratories, San Francisco, CA), anti-very late antigen (VLA)-1, 3, 5 (Immunotech), and negativeness against immunostaining of VIII factor (Dako, CA).

### Example 2: Isolation of mRNA from human cultured mesangial cells

At the sixth passage, total RNA was isolated from human mesangial cells using guanidine isothiocyanate (GTC) method. The confluent culture of the mesangial cells in the medium containing serum of the cells of Example 1 was washed with phosphate buffer saline (PBS), and dissolved in 5.5 mM GTC solution. DNA was removed by passing through an 18-gauge needle. Nuclei and other cell debris were precipitated by centrifugation at 5,000X g for 90 sec. Supernatant was carefully loaded on the layer of cesium trifluoroacetate (CsTFA) and centrifuged at 125,000X g at 15°C for 24 hours. RNA pellet was dissolved in TE buffer. Poly(A)⁺ RNA was isolated using oligo dT cellulose column (Pharmacia).

### Example 3: Construction of 3'-directed cDNA library

cDNA was synthesized using the vector primer based on pUC19 (Norrander J. et al., Gene, 26, 101-106, 1983) with poly (A)⁺ RNA as a template. This vector primer DNA comprised the HincII end and the PstI end with a T tale, and dam-methylated at the MboI site (GATC). After synthesizing the second strand, the cDNA sequence and the single BamHI site in LacZ gene of the vector were digested with MboI and BamHI, respectively, and circularization and ligation were conducted at the low DNA concentration. A portion of the ligation mixture was transformed to *E. coli.* The obtained transformants were randomly selected and individually dissolved by simply heating. The inserted sequence of cDNA was amplified by the paired PCR using primers (5'-TGTAAAACGACGGCCAGT-3'/SEQ ID NO: 3 and 5'-ACCATGATTACGCCAAGCTTG-3'/SEQ ID NO: 4) flanking the pUC19 cloning site. The obtained short double stranded DNA was used for the cycle sequence determination reaction and analyzed by an automatic sequencer.

### Example 4: Isolation of genes expressed specifically in mesangial cells

In order to identify genes expressed specifically in mesangial cells, the present inventor conducted large scale DNA sequencing and data processing by computers. Transcripts in the various different cells and organs could be simultaneously compared (Y. Yasuda et al., Kidney International 53:154-158, 1998; K. Matsubara et al., Gene. 135, 265 (1993); K. Okubo et al., Nat. Gen. 2, 173 (1992)). Large scale DNA sequencing of the 3'-domain cDNA library of human cultured mesangial cells was conducted, and randomly selected 1836 clones were sequenced for their partial sequences. The sequence homology among clones was mutually compared, and further compared with that in DNA data bank GenBank using FASTA program. mRNA from various organs and cells were analyzed using dot blot to select clones expressed specifically in mesangial cells. As a result, some clones detected at extremely high frequency in the mesangial cell cDNA library were obtained.

### Example 5: Cloning of the full-length cDNA by 5'RACE

The following experiment was carried out using "5'-Full RACE Core Set" (Takara shuzo, Co. Ltd.). To a 0.5 mL microtube were added 4 0 µL of human poly (A)⁺ RNA prepared from human mesangial cell culture (0.5 µg/µL), 1.5 µL of 10X RT buffer, 0.5 µL of RNase inhibitor (40 U/µL), 1 µL of AMV Reverse Transcriptase XL (5U/µL) and 1 µL of a 5'-terminal phosphorylated RT primer (5'-pTCAGAGAGGTCATTC/SEQ ID NO: 5, 200 pmol/µL), and the total volume was adjusted to 15 µL with 7 µL of RNase free dH₂O. The reaction mixture was placed in the "Takara PCR Thermal Cycler" (Takara shuzo, Co. Ltd.) and incubated for 10 minutes at 30°C, for 60 minutes at 50°C, for 2 minutes at 80°C and for 10 minutes at 4°C to obtain the first-strand cDNA.

Fifteen microliters of the reaction solution was added to a 0.5 mL microtube containing 15 µL of 5X hybrid RNA denaturation buffer and 45 µL of H₂O. After adding 1 µL of RNaseH, a reaction was conducted for one hour at 30°C. After the reaction, 150 µL of ethanol was added to the reaction mixture, which was then cooled for 30 minutes at -70°C. The mixture was centrifuged, and the supernatant was removed to collect the pellet.

To the pellet, 8 µL of 5X RNA (ssRNA) ligation buffer, 20 µL of 40% PEG #600 and 12 µL of H₂O were added and mixed well. One microliter of T4 ligase was then added to the mixture, which was then incubated for 15 hours at 16°C to obtain cyclized single-stranded cDNA.

The cyclized single-stranded cDNA thus obtained was used as template after 1:10 dilution with TE buffer, followed by primary PCR. The reaction conditions were 5 µL of 10X LA PCR buffer II (Mg²⁺plus), 8 µL of dNTP mixture (2.5 mM), 0.5 µL of primary PCR S1 primer (5'-TCATTGATGGGTCCTCAA/SEQ ID NO: 6, 20 pmol/µL), 0.5 µL of primary PCR A1 primer (5'-AGATTCTTGAGCTCAGAT/SEQ ID NO: 7, 20 pmol/µL), 0.5 µL of TaKaRa LA TaqTM (5U/µL) , and the total volume of the mixture was adjusted to 50 µL with sterilized water. The mixture was placed in the "Takara PCR Thermal Cycler", heated for 3 minutes at 94°C, and subjected to 25 cycles of the reaction as follows: for 30 seconds at 94°C, for 30 seconds at 60°C and for 2 minutes at 72°C.

One microliter of the primary reaction solution was used as template, to which 5 µL of 10X LA PCR TM buffer II (Mg²⁺plus), 8 µL of dNTP mixture (2.5 mM), 0.5 µL of the secondary PCR S2 primer 5'-AATGGTGGCATAAACATG/ SEQ ID NO: 8, 20 pmol/µL), 0.5 µL of the secondary PCR A2 primer 5'-ACAGACAAATTGAACTTC/ SEQ ID NO:9, 20 pmol/µL), and 0.5 µL of TaKaRa LA TaqTM (5U/µL) were added, and the total volume of the mixture was adjusted to 50 µL with sterilized water. The mixture was placed in the "Takara PCR Thermal Cycler", and subjected to 30 cycles of the reaction for 30 seconds at 94°C, for 30 seconds at 60°C and for 2 minutes at 72°C.

After the appearance of the band was verified by 0.75% agarose gel electrophoresis, 1 µL of the reaction solution was subcloned using the "Original TA cloning Kit" (Invitrogen). The nucleotide sequence of the gene fragment inserted into the plasmid recovered was determined by the dideoxy termination method.

The result showed that the nucleotide sequence obtained contained the region encoding the N-terminal portion of the gene product and about 50 additional nucleotides as the 5'-untranslated region. The predicted position of the initiation codon ATG corresponded to that in the consensus sequence, giving the longest ORF (fulfilling "the first ATG rule"). The amino acid sequence consisting of 950 amino acid residues based on the longest ORF found in the cDNA sequence was determined as the deduced amino acid sequence of the gene product. The protein having this amino acid sequence was designated Meg-1 by the present inventor. The nucleotide sequence of the meg-1 cDNA is shown in SEQ ID NO: 1 and the deduced amino acid sequence thereof are shown in SEQ ID NO: 2.

### Example 6: Functional analysis of the mesangium-specific gene (1)

Amino acid homology search of the SwissProt database with FASTA program confirmed that Meg-1 is a novel protein. It also verified that protein serine/threonine phosphatase 4 related protein PP4_{R1} shows a homology. Meg-1 has 97.9% homology to PP4_{R1} in the amino acid sequence and 98.3% in the nucleic acid sequence of cDNA. In addition, a nucleotide sequence for a portion of the amino acid sequence shown as SEQ ID NO:2 (173 amino acid residues extending positions 778 to 950) is 99.4% identical to the nucleotide sequence (HSU79267) of a cDNA submitted as an EST derived from fetal brain EST, and 100% identical in the amino acid sequence. This clone is derived from Soares library 1NIB from IMAGE consortium, and the EST is merely a partially determined sequence whose function and bodymap is totally unknown.

Subsequently, a motif search was conducted on the amino acid sequence of Meg-1. PSORT WWW Server (http://psort.nibb.ac.jp:8800/) and MOTIF (http://www.motif.genome.ad.jp/) were used for the search. The result revealed that Meg-1 contains several phosphorylation motifs. Specifically, the presence of the motifs for phosphorylation by the kinases shown below has been identified. Additionally, six N-glycosylation sites were verified. Furthermore, the presence of a nuclear localization signal was also suggested. The nuclear localization was predicted to be 52.2%.
cAMP/cGMP dependent protein phosphorylation site: 1
casein kinase II phosphorylation site: 22
protein kinase C phosphorylation site: 11
tyrosine kinase phosphorylation site: 2

Furthermore, based on these facts, it was confirmed that the above-predicted amino acid sequence with 950 amino acid residues corresponds to the amino acid sequence of Meg-1. The theoretical pI of the protein with this amino acid sequence is 4.49.

### Example 7: Functional analysis of Meg-1 (2) - tissue distribution

Northern blot analysis of Meg-1 was performed as described in the following. The positive clone insert of the 3' directed cDNA library (Example 3) was labeled with RI by random DNA labeling and was used as a probe. Poly (A)⁺RNA (2 )µg) isolated from the below-mentioned cells to be used as samples was separated on a 1% agarose gel containing 2.2 M formamide and was transferred onto a nitrocellulose filter. The filter was hybridized in Rapid Hyb solution (Amersham, Arlington Heights, IL). After hybridization, it was washed with final stringency of 0.1X SSPE/0.1% SDS at 60°C.

Samples for Northern blot analyses of multiple human primary culture cells and tissues, and for Northern blot .analysis of human cancer cell lines were purchased from Clontech (Palo Alto, CA). As samples of primary culture cells, 2 µg of poly (A) ⁺RNA from primary culture cells of mesangial cells, human dermal epithelial cells, human renal cortical epithelial cells, human endothelial cells of the umbilical vein, and human smooth muscle cells were used. For Northern blot analysis of human cancer cell lines, 2 µg of poly (A) ⁺RNA derived from promyelocytic leukemia HL-60, HeLa cells S3, chronic myeloid leukemia K-562, lymphoblastic leukemia MOLT-4, Burkitt's lymphoma Raji, adenocarcinoma of the large intestine SW480, lung cancer A549, and melanoma G361 were used as samples. For Northern blot analysis of the tissues, 2 µg of poly (A) ⁺RNA derived from the heart, brain, placenta, lung, liver, skeletal muscles, kidney and pancreas were used as samples. Hybridization and washing were carried out as described above. The results are as indicated in Tables 1 to 3.

**Table 1**

| | |
|---|---|
| Primary culture cells | |
| Human mesangial cells | + + + |
| Human dermal fibroblast cells | + + |
| Human renal cortical epithelial cells | + |
| Human endothelial cells of the umbilical vein | ± to + |
| Human smooth muscle cells | ± to + |

**Table 2**

| | |
|---|---|
| Human cancer cell lines | |
| Promyelocytic leukemia HL-60 | + + |
| HeLa cells S3 | + + + |
| Chronic myeloid leukemia K-562 | + + + |
| Lymphoblastic leukemia,MOLT-4 | + + + |
| Burkitt's lymphoma Raji | - to ± |
| Adenocarcinoma of the large intestine SW480 | + + + |
| Lung cancer A549 | + + + |
| Melanoma G361 | + + |

**Table 3**

| Human tissues | |
|---|---|
| Heart | + + + |
| Brain | + + |
| Placenta | + + + |
| Lungs | - |
| Liver | + |
| Skeletal muscle | + + |
| Kidney | + + |
| Pancreas | + + |

In the Northern blot analysis using Meg-1 cDNA as a probe, a single transcription product (approximately 4.5 kb) was found in the cultured mesangial cells. One of the characteristics in the human primary cell culture was that the Meg-1 gene is especially highly expressed in mesangial cells. In other primary cell cultures, expression in renal cortical epithelial cell and dermal fibroblast are observed, and slight expression in endothelial cell of the umbilical vein and smooth muscle cells was observed. Furthermore, when those in tissues are compared, high expression of Meg-1 was observed in the heart and placenta followed by the expression in the brain, skeletal muscle, kidney, and pancreas. Further, expression in the liver was weak, and no expression could be detected in the lung. Among cultured cancer cell lines, strong expression was observed in HeLa cells S3, chronic myeloid leukemia K-562, lymphoblastic leukemia MOLT-4, adenocarcinoma of the large intestine SW480, and lung cancer A549. It was also expressed in promyelocytic leukemia HL-60 and melanoma G361. Almost no expression was observed in Burkitt's lymphoma Raji.

### Example 8: Functional analysis of a Meg-1 (3) -in situ hybridization

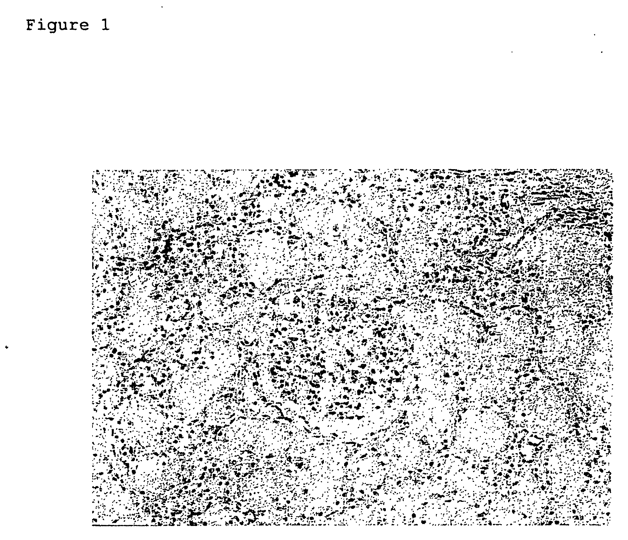

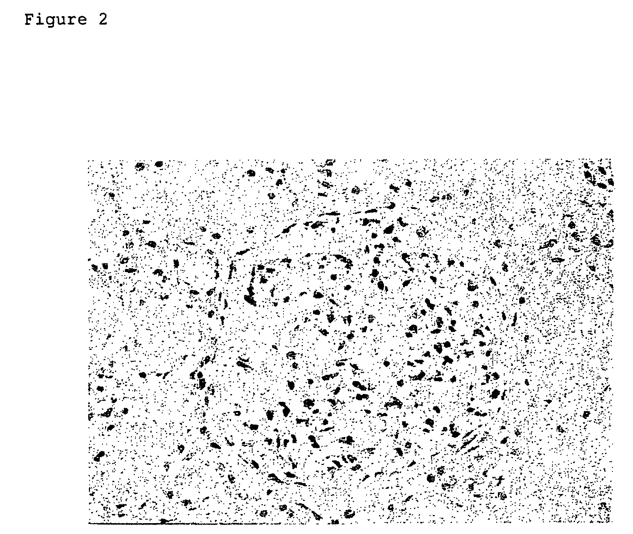

Meg-1 mRNA expression was evaluated in a tissue by in situ hybridization using human renal tissue obtained from the kidney of a normal healthy person. *In situ* hybridization was conducted following known methods (Kidney Int. 52:111 (1997)). Nucleotide sequence corresponding to position 2879 to 2912 of the human Meg-1 (SEQ ID NO: 10) was used as the probe. As shown in Figures 1 and 2, it was confirmed that cells in the mesangium region of glomeruli are Meg-1 mRNA positive.

### Example 9: Rat Meg-1

Acquisition of a rat homologue of Meg-1 was attempted.

Total RNA was collected from rat kidney mesangial cells and cDNA was synthesized. Using this cDNA as template, PCR was performed using various degenerative primers based on the nucleotide sequence of human Meg-1. The degenerative primers were designed in the portions corresponding to the following region of human Meg-1:
Sense strand: nucleotide positions 1-18;
Anti-sense strand: nucleotide positions 2852-2872.

An amplified product with the predicted size was cloned, and its nucleotide sequence was determined. Furthermore, the 3' terminus was partially sequenced using the marathorn method (the method used for isolation and identification of megsin) . The nucleotide sequence obtained is shown in SEQ ID NO: 11, and the deduced amino acid sequence is shown in SEQ ID NO: 12.

As a result, a rat Meg-1 homologue whose nucleotide sequence has not been reported so far was isolated. Rat Meg-1 shares a highly conserved structure with human Meg-1, 74.3% at the gene level and 86.3% at the protein level.

In addition, localization of rat Meg-1 in the rat cell and tissue was analyzed using the same method as described in Example 7 with the exception of the sample and probes used. The results were as follows:

| | |
|---|---|
| Brain | + |
| Lung | +++ |
| Heart | + |
| Liver | + |
| Spleen | ++ |
| Kidney | ++ |
| Smooth muscle | - to + |
| Mesangial cells | +++ |
| Renal epithelial cells | - to + |
| Renal endothelial cells | - to + |

Since Meg-1 is highly expressed in the kidney (mesangial cells in particular) over the species including both humans and rats, it is possibly one of the functional genes that are involved in the mesangial cell function.

### Example 10: Specific expression of Meg-1 in the mesangial nephritis-induced glomerulus

It is known that mesangial nephritis can be induced in rat by administering a monoclonal antibody raised against the rat Thyl antigen (Yagi M, Yamamoto T, Nagano N, Kato S, Kusaka M, Kawasaki K, Yaoita E, Kihara T: Transient expression of type I collagen in glomeruli with anti-Thy-1 antibody-induced mesangial proliferative lesions. Pathol Int 45: 409-414, 1995). By this method, mesangial nephritis was induced in the rat glomerulus, and Meg-1 expression was detected there using Northern blot analysis.

Anti-rat Thy1 monoclonal antibody was prepared by using a hybridoma line producing the antibody (purchased from European Collection of Animal Cell Cultures (Sulisbury, UK)) (Salant DJ, Darby C, Couser WG: Experimental membranous glomerulonephritis in rats. J Clin Invest 66: 71-81, 1980). In brief, the hybridoma cells were cultured in RPMI-1640 medium containing 10% fetal bovine serum, and 1x 10⁶ cells suspended in 0.5 ml of RPMI-1640 were inoculated into the peritoneal cavities of 10-week-old male Balb/c mice which were injected with incomplete Freund's adjuvant intraperitoneally 7 days earlier. Within 2 weeks after the hybridoma injection, ascites was obtained from the mice. The ascitic fluid was centrifuged to remove insoluble materials, and the supernatant was pooled. Then, the pooled ascites was dialyzed against PBS, and the IgG-rich fraction was precipitated with 50% saturated ammonium sulfate. The crude antibodies were applied to HiTrap Protein A column (Pharmacia Biotech, Tokyo, Japan) to purify IgG. IgG adsorbed on the column was eluted into the elution buffer (0.1 M sodium citrate, pH 5), and was dialyzed against PBS before use.

Meg-1 mRNA expression in rat glomeruli where mesangial nephritis was induced was detected as follows. All animal experimentation was conducted in accord with Guide for Animal Experimentation, Faculty of Medicine, University of Tokyo. After male Wistar rats, 6 weeks old (purchased from Charles River Japan (Yokohama, Japan)) were bred for 1 week, they received a 1.2 mg/Kg body wt intravenous injection of IgG1 mouse monoclonal anti-Thyl antibodies (OX7) or vehicle (control). Before the administration, and 2, 4, 7, 14, and 28 days after the administration, kidneys were excised from six respective rats and cut into small pieces. Glomeruli were separated from the small pieces through a sieve. Total RNA was isolated from these glomeruli and subjected to Northern blot analysis. A fragment having the sequence of 841 to 1979^{th} nucleotide (SEQ ID NO: 13) of rat Meg-1 was used as the probe. This region is suited for a probe because of low homology to subunit A (regulatory subunit) of protein phosphatase 2A (PP2A). The probe was prepared by subcloning a fragment that was amplified by PCR using rat mesangial cell cDNA as the template into pGEM-T easy and digesting it by EcoRI.

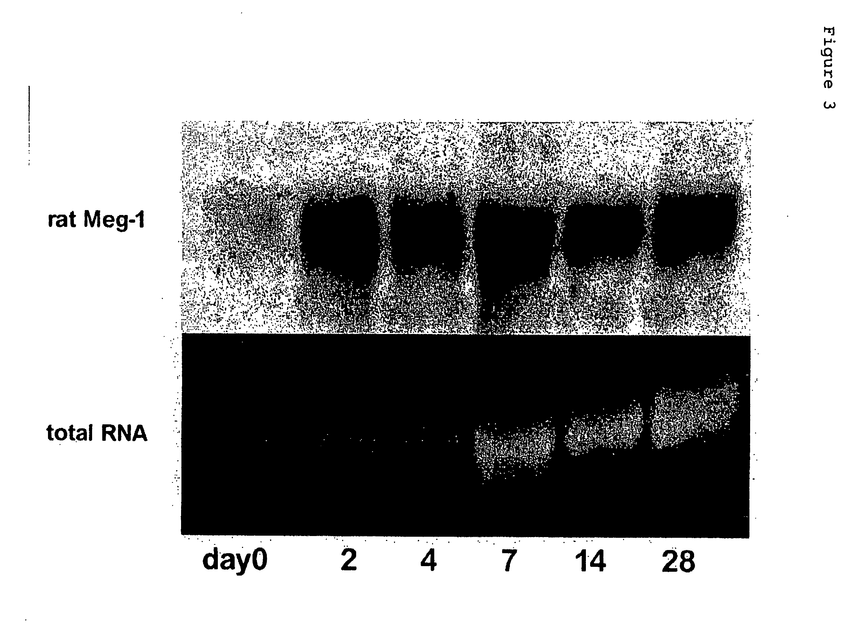

The result of the Northern blot analysis confirmed that Meg-1 expression remarkably increases in the glomeruli where mesangial nephritis was induced by anti-Thyl antibody (Figure 3).

### Industrial Applicability

The present invention provides a DNA expressed at high frequency in mesangial cells, a protein encoded by the DNA, an antibody binding to the protein, etc. These are supposed to be closely related to mesangial cell-specific bioactivity, and useful for, for example, identifying mesangial cells, and detecting abnormalities in mesangial cells. Moreover, this protein would be helpful for clarifying the functions of mesangial cells and in turn, for investigating causes of diseases relating to mesangial cells. This invention is expectedly applicable to the treatment and diagnosis, and such of diseases relating to mesangial cells, such as mesangial nephritis.

Specifically, the onset or progress of glomerulonephritis may be regulated, for example, by artificially adjusting Meg-1. Alternatively, quantification of mRNA and protein of Meg-1 in the body fluid and mesangial cells may enable diagnosis of renal diseases such as glomerulonephritis. In glomerulonephritis, functional abnormality is seen in the mesangium region, causing proliferation of mesangial cells or acceleration of matrix production from the cells . The possibility that Meg-1 is involved in these diseases is great.

Meg-1 of this invention and MEGSIN, which was previously reported by the present inventor, share common characteristics with regard to high levels of expression in the mesangial cells. However, the possibility that Meg-1 of this invention is a nuclear protein having nuclear localization signal is suggested because of a homology to a phosphatase, whereas MEGSIN is a protein that is homologous to the SERPIN superfamily, which is a protease inhibitor. In addition, the observation that Meg-1 of this invention is expressed in a variety of tissues is different from the characteristic of MEGSIN, which is expressed specifically in mesangial cells. Therefore, Meg-1 of this invention may possibly be an important protein that supports the function of mesangial cells. This invention, which has elucidated the existence of such an important protein, has great significance.

## Claims

1. A polynucleotide selected from the group consisting of (a) to (d) below:
(a) a polynucleotide comprising a protein-coding region of the nucleotide sequence of SEQ ID NO: 1;
(b) a polynucleotide encoding a protein comprising the amino acid sequence of SEQ ID NO: 2;
(c) a polynucleotide comprising the amino acid sequence of SEQ ID NO: 2 in which one or more amino acids are substituted, deleted, inserted and/or added, said polynucleotide encoding a protein functionally equivalent to a protein comprising the amino acid sequence of SEQ ID NO: 2; and
(d) a polynucleotide hybridizing to a DNA comprising the nucleotide sequence of SEQ ID NO: 1 under stringent conditions, said polynucleotide encoding a protein functionally equivalent to a protein comprising the amino acid sequence of SEQ ID NO: 2.

2. A protein encoded by the polynucleotide of claim 1.

3. A vector comprising the polynucleotide of claim 1.

4. A transformant carrying the polynucleotide of claim 1 or the vector of claim 3.

5. A method for producing the protein of claim 2, said method comprising culturing the transformant of claim 4 and recovering an expression product.

6. An oligonucleotide having a chain length of at least 15 nucleotides, said oligonucleotide comprising a nucleotide sequence complementary to the nucleotide sequence of SEQ ID NO: 1 or to a complementary strand thereof.

7. A method for detecting a polynucleotide comprising the nucleotide sequence of SEQ ID NO: 1, said method comprising contacting the oligonucleotide of claim 6 with a test sample and observing hybridization of said oligonucleotide.

8. A method for synthesizing a polynucleotide comprising the nucleotide sequence of SEQ ID NO: 1, said method comprising contacting the oligonucleotide of claim 6 with a test sample and synthesizing the complementary strand using said oligonucleotide as a primer.

9. A reagent for detecting mesangial cells, said reagent comprising the oligonucleotide of claim 6.

10. A method for detecting mesangial nephritis, said method comprising measuring an expression level of the polynucleotide of claim 1 in a biological sample, and comparing said level with that obtained from a normal sample.

11. The method of claim 10, wherein said biological sample is a mesangial cell.

12. The method of claim 10, wherein said expression level is measured using, as an indicator, an mRNA comprising a nucleotide sequence selected from the nucleotide sequence of SEQ ID NO: 1.

13. The method of claim 10, wherein said expression level is measured using, as an indicator, the protein of claim 2 or a fragment thereof.

14. An antisense polynucleotide against the polynucleotide of claim 1 or a portion thereof.

15. An antibody recognizing the protein of claim 2.

16. The antibody of claim 15, recognizing a portion of a protein comprising an amino acid sequence selected from the amino acid sequence of SEQ ID NO: 2.

17. An immunological assay for measuring the protein of claim 2 or a fragment thereof based on immunological binding of the antibody of claim 15 to the protein of claim 2 or a fragment thereof.

18. A reagent for immunological assay of the protein of claim 2 or a fragment thereof, said reagent comprising the antibody of claim 15.

19. A transgenic non-human vertebrate, wherein an expression level of a gene encoding Meg-1 is altered.

20. The transgenic non-human vertebrate of claim 19, wherein said non-human vertebrate is a mouse.

21. The transgenic non-human vertebrate of claim 20, wherein said transgenic non-human vertebrate is a knockout mouse in which the expression of a gene encoding Meg-1 is inhibited.
